# EUROPÄISCHE PATENTSCHRIFT

(11) **EP 3 280 472 B1**
(45) Veröffentlichungstag und Bekanntmachung des Hinweises auf die Patenterteilung: **27.02.2019**
(21) Anmeldenummer: 16727284.8
(22) Anmeldetag: 01.06.2016
(51) Int. Cl.: A61M 5/20, A61M 5/315

(54) **INJEKTIONSVORRICHTUNG MIT GEWINDE MIT EINER VARIABLEN STEIGUNG**
INJECTION DEVICE HAVING A THREAD HAVING A VARIABLE PITCH
DISPOSITIF D'INJECTION POURVU D'UN FILETAGE À PAS VARIABLE

(30) Priorität: 23.06.2015 CH 9042015
(43) Veröffentlichungstag der Anmeldung: 14.02.2018
(73) Patentinhaber: TecPharma Licensing AG, 3401 Burgdorf (CH)
(72) Erfinder: HOSTETTLER, Patrick, 3415 Hasle (CH); TSCHIRREN, Markus, 3400 Burgdorf (CH)
(86) Internationale Anmeldenummer: PCT/CH2016/000084
(87) Internationale Veröffentlichungsnummer: WO 2016/205961

(56) Entgegenhaltungen:
- EP-A1- 2 692 377
- EP-A1- 2 881 131
- WO-A1-2014/166887
- WO-A1-2014/166914
- WO-A1-2015/032455
- WO-A1-2015/055642
- WO-A1-2016/071483

## Beschreibung

Die Erfindung betrifft eine Injektionsvorrichtung zum Verabreichen eines flüssigen Produkts insbesondere eines hoch viskosen Medikaments. Im Besonderen betrifft die Erfindung eine Antriebs- und Signalvorrichtung sowie ein Verfahren für eine solche Injektionsvorrichtung.

Der Begriff "Medikament" oder Produkt umfasst hier jede fließfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament oder Produkt umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe.

Aus dem Stand der Technik, WO201307800 ist ein Injektionsgerät, insbesondere ein Autoinjektor mit automatischem Einstechvorgang und einem automatischen Ausschüttvorgang und einer Signalvorrichtung bekannt. Wobei die Energie zum Einstechen, Ausschütten und für die Signalvorrichtung von der Einstech-und Ausschüttfeder geliefert wird. Während der Ausschüttung ist die Einstech-und Ausschüttfeder an eine Antriebsmutter gekoppelt, wodurch eine Gewindeartige Kolbenstange angedreht und in axiale Richtung verschoben wird. Dabei nimmt das Federkraftmoment während der Ausschüttung und insbesondere gegen Ende der Ausschüttung ab. Das heisst gegen Ende der Ausschüttung wird mit weniger Kraft ausgeschüttet als am Anfang der Einstech- bzw. Ausschüttbewegung.

Die Patentanmeldung WO 2015/032455 (Tecpharma) offenbart eine Antriebs- und Dosiervorrichtung für eine Injektionsvorrichtung zum Verabreichen eines Medikaments. Die Antriebs- und Dosiervorrichtung umfasst ein Gehäuse, ein Betätigungsglied, ein Vortriebsglied dessen distales Ende vorgesehen ist, auf einen Kolben eines an der Antriebsvorrichtung befestigten Produktbehälters zu wirken, und eine Gewindestange. Durch Drehung der Gewindestange relativ zum Gehäuse und dem Vortriebsglied kann eine Feder das Vortriebsglied um einen Ausschütthub in distale Richtung verschieben, der proportional zu dem Drehwinkel der Gewindestange ist. Durch wahlweises Sperren und Freigeben der Gewindestange, was durch Betätigen des als Betätigungsknopf ausgestalteten Betätigungsglieds bewirkt werden kann, kann die Bewegung des Vortriebsglieds relativ zu dem Gehäuse gesteuert werden.

Es ist eine Aufgabe der Erfindung, eine Injektionsvorrichtung anzugeben, die eine Einrichtung zum Ausschütten von flüssigen Medikamenten aufweist.

Die Aufgabe wird mit dem Autoinjektor nach Anspruch 1 gelöst. Vorteilhafte Weiterentwicklungen ergeben sich aus den abhängigen Ansprüchen, der Beschreibung und den Figuren.

Autoinjektor zur Ausschüttung eines flüssigen Produkts, insbesondere eines hochviskosen Medikaments, umfassend: ein Gehäuse und einen in dem Gehäuse angeordneten Produktbehälter, der einen verschiebbaren Kolben aufweist, wobei der Kolben zur Ausschüttung des in dem Produktbehälter enthaltenen Produkts in eine Ausschüttrichtung verschiebbar ist,
ein Vortriebsglied , das während der Produktausschüttung auf den Kolben wirkt, und eine erste Feder , die vorgespannt ist, damit das Produkt aus dem Produktbehälter durch Verschieben des Vortriebsglieds und des Kolbens ausgeschüttet werden kann,
gekennzeichnet durch
ein Rotationsglied welches operativ mit dem Vortriebsglied gekoppelt ist, wobei die erste Feder so auf das Rotationsglied wirkt, dass zur Produktausschüttung das Rotationsglied in Rotation versetzt wird, wobei das Rotationsglied oder das Vortriebsglied ein Gewinde mit einer variablen Steigung aufweist.

Entweder das Vortriebsglied mindestens ein Gewindesegment aufweist und mit dem Rotationsglied in Gewindeverbindung ist oder das Rotationsglied mindestens ein Gewindesegment aufweist und mit dem Vortriebsglied in Gewindeverbindung ist.

Eine Flanke des mindestens einen Gewindesegments des Vortriebsglieds oder des Rotationsglieds verschiedene Steigungswinkel aufweist.

Das Rotationsglied einen Gewindeeinlauf mit einem Axialabschnitt aufweist und zwischen dem Vortriebsglied und dem Kolben ein Abstand insbesondere ein Beschleunigungsweg ist, wobei der Axialabschnitt grösser als der Beschleunigungsweg ist.

Das Gewinde mit der variablen Steigung, zumindest einen Bereich mit einer stetigen Steigungsvariation aufweist oder/ und die Steigungsvariation des Gewindes mit der variablen Steigung zumindest bereichsweise unstetig verläuft.

Das Gewinde mit der variablen Steigung eine degressive Gewindesteigung aufweist, wodurch der Abfall des Federdrehmoments während dem Ausschütten kompensiert werden kann,

Bei einer Schraubbewegung zwischen der Gewindestange und dem mindestens einem Gewindesegment des Vortriebsglieds , die Flanke des mindestens einem Gewindesegments an dem Gewinde mit der variablen Steigung der Gewindestange geschraubt wird, wobei unterschiedliche Bereiche der Flanke das Gewinde mit der variablen Steigung berühren.

Das Vortriebsglied eine erste Gewindeverbindung mit dem Rotationsglied und eine zweite Gewindeverbindung mit dem Gehäuse oder einem gehäusefesten Element aufweist.

Die erste Feder eine Spiralfeder ist,

Die Gewindestange axialfest in dem Gehäuse gelagert ist und so mit der ersten Feder gekoppelt ist, dass ein Entspannen der ersten Feder zur Rotation der Gewindestange führt und dass das Vortriebsglied rotationsfest gegenüber dem Gehäuse ist.

In einer Lagerposition die Axialkräfte, welche insbesondere durch eine Gewindeübersetzung aus dem Drehmoment der Feder entstehen gering gehalten werden, indem in der Lagerposition das Gewinde mit der variablen Steigung an einem Ort mit grosser Steigung in Gewindeverbindung ist.

Ein Halteelement zumindest einen axial gerichteten Arm aufweist und an dem zumindest einen Arm ein erstes Eingriffselement und ein zweites Eingriffselement angebracht sind, und wobei das erste Eingriffselement in eine Ausnehmung des Vortriebsglieds lösbar eingreift, wodurch das Vortriebsglied axialfest mit dem Halteelement gekoppelt ist, wobei die Kopplung zwischen dem Vortriebsglied und dem Halteelement gelöst ist, wenn das Halteelement aus dem Eingriff mit dem Vortriebsglied ist, wobei durch den Eingriff das Vortriebsglied daran gehindert wird, sich relativ zu dem Halteelement in Ausschüttrichtung zu bewegen, wobei dieser Eingriff des ersten Eingriffselements für die Produktausschüttung lösbar ist, so dass die erste Feder das Vortriebsglied relativ zu dem Halteelement in die Ausschüttrichtung antreiben kann.

Das Vortriebsglied mittels der ersten Feder relativ zu dem Halteelement in die distale Richtung bewegbar ist, wenn das erste Eingriffsglied aus dem Eingriff mit dem Vortriebsglied und das zweite Eingriffsglied in dem Eingriff mit der Nadelschutzhülse oder einem Schaltmodul ist,

Das Halteelement mit einem Vortriebsglied und/ oder mit dem Schaltmodul in Eingriff ist.

Verfahren zur Mehrfachverwendung eines Halteelements in einem Autoinjektor enthaltend zumindest zwei der folgenden Schritte:
Halten des Vortriebsglieds mittels des Halteelements
Axiale Bewegung des Halteelements zur Erzeugung eines Startklicksignals
Axiale Bewegung des Halteelements zur Erzeugung eines Endklicksignals

### Weitere bevorzugte Aspekte der Erfindung können sein:

Das Schaltmodul weist eine Schalthülse und Sperrhülse auf, wobei die Sperrhülse ein unidirektional wirkendes Verriegelungsglied aufweist, welches in die Schalthülse eingreift, wobei die Schalthülse bei ihrer Bewegung relativ zu dem Gehäuse in die proximale Richtung die Sperrhülse über das Verriegelungsglied mitnimmt und bei ihrer Bewegung relativ zu dem Gehäuse in proximale Richtung relativ zu der Sperrhülse in eine weitere Sperrposition verschoben wird, in der das Verriegelungsglied eine Bewegung der Schalthülse relativ zu der Sperrhülse in die proximale Richtung sperrt, wobei das Halteelement mit zumindest einem zweiten Eingriffselement in die Sperrhülse greift.

Das Schaltmodul, welches kinematisch oder/und geometrisch zwischen einer zweiten Feder und der Nadelschutzhülse angeordnet ist, wobei das Schaltmodul, von der Nadelschutzhülse in die proximale Richtung mitgenommen wird, wenn die Nadelschutzhülse aus ihrer Ausgangsposition in die proximale Richtung verschoben wird.

Das Halteelement, mit dem ersten Eingriffselement, vor dem Auslösen der Produktausschüttung in das Vortriebsglied eingreift, wodurch das Vortriebsglied daran gehindert wird, sich relativ zu dem Halteelement in Ausschüttrichtung zu bewegen, wobei dieser Eingriff des ersten Eingriffselements für die Produktausschüttung lösbar ist, so dass die erste Feder das Rotationsglied antreiben kann und, das Vortriebsglied relativ zu dem Halteelement in die Ausschüttrichtung verschieben kann.

Der Autoinjektor einen Startsignalanschlag und der zweiten Feder, welche auf das Halteelement eine entgegen der Ausschüttrichtung wirkende Federkraft ausübt aufweist, wobei das Halteelement mit dem Vortriebsglied gekoppelt ist, und wobei die axialfeste Kopplung zwischen Halteelement und Vortriebsglied lösbar ist und das Halteelement mittels der zweiten Feder entgegen der Ausschüttrichtung und relativ zu dem Vortriebsglied oder/und dem Gehäuse beschleunigbar ist,

Das Halteelement mit dem zweiten Eingriffselement, welches durch die Ausrückbewegung des ersten Eingriffselements aus dem Vortriebsglied in einen axialfesten Eingriff mit der Nadelschutzhülse oder dem Schaltmodul, insbesondere in die Sperrhülse bewegbar ist, wobei das von der axialfesten Kopplung mit dem Vortriebsglied entkoppelte, von der zweiten Feder beschleunigte Halteelement die Sperrhülse in einen Startsignalanschlag bewegt und an dem Slartsignalanschlag anschlägt, wodurch ein akustisches und/oder taktiles Startsignal erzeugt wird,

Das Vortriebsglied das zweite Eingriffsglied daran hindert, aus dem Eingriff mit der Sperrhülse auszurücken, wenn sich das Vortriebsglied relativ zu dem Halteelement in distale Richtung bewegt, wobei das Vortriebsglied am Ende des Ausschütthubs (H_{A}) dem zweiten Eingriffsglied erlaubt, aus dem Eingriff mit der Sperrhülse auszurücken, wodurch das Halteelement von der zweiten Feder entgegen die Ausschüttrichtung beschleunigt wird und an einen Endsignalanschlag anschlägt, wodurch ein akustisches und/oder taktiles Endsignal erzeugt wird.

Der Endsignalanschlag von dem Gehäuse oder einem zumindest axialfest, vorzugsweise auch drehfest, mit dem Gehäuse verbundenen Element, wie z. B. der Mechanikhalter ist.

Das Halteelement mit dem zweiten Eingriffsglied, welches durch die Ausrückbewegung des ersten Eingriffsglieds aus dem Vortriebsglied in einen axialfesten Eingriff mit der Nadelschutzhülse oder dem Schaltmodul bewegbar ist, wobei das erste Eingriffsglied und das zweie Eingriffsglied so aufeinander abgestimmt sind, dass das zweite Eingriffsglied bereits axialfest in die Nadelschutzhülse oder das Schaltmodul eingreift, wenn das erste Eingriffsglied noch nicht vollständig aus dem Eingriff mit dem Vortriebsglied gelöst ist.

Eine auf die zweite Feder wirkende Nadelschutzhülse, welche für die Auslösung der Produktausschüttung aus ihrer Ausgangsposition relativ zu dem Gehäuse und entlang der Längsachse des Autoinjektors in die proximale Richtung, insbesondere um einen Betätigungshub verschiebbar ist, wodurch die zweite Feder gespannt und insbesondere die Produktausschüttung ausgelöst wird.

Die Nadelschutzhülse nach der Produktausschüttung, insbesondere wenn das Vortriebsglied um den Ausschütthub in die distale Richtung verschoben ist, von der zweiten Feder relativ zu dem Gehäuse in die distale Richtung, insbesondere um einen Nadelschutzhub (H_{S}) in eine Nadelschutzposition verschiebbar ist, in der die Nadelschutzhülse distal über die Nadelspitze einer injektionsnadel des Produktbehälters steht.

Ein Verriegelungsglied, welches die Nadelschutzhülse in ihrer Nadelschutzposition relativ zu dem Gehäuse gegen Zurückschieben in die proximale Richtung verriegelt, insbesondere zumindest so verriegelt, dass die Nadelspitze nicht aus dem distalen Ende der Nadelschutzhülse hervortreten kann.

Der erfindungsgemässe Autoinjektor weist ein Gehäuse und ein in dem Gehäuse angeordneten Produktbehälter auf. Bei dem Produktbehälter kann es sich insbesondere um eine Spritze oder einer Karpule handeln, welche einen Behälterkörper aufweist, an dessen distalem Ende eine Injektionsnadel entweder fest angeordnet ist oder eine Injektionsnadel angebracht werden kann. Der Behälterkörper umgibt einen Kolben, der in Bezug auf den Spritzenkörper verschiebbar ist und für die Produktausschüttung zu dem distalen Ende hin verschoben wird, wodurch das zwischen dem Kolben und der Injektionsnadel angeordnete flüssige Produkt, insbesondere ein hochvisköses Medikament, durch die Injektionsnadel aus dem Produktbehälter ausgeschüttet wird. Der Spritzenkörper kann an seinem proximalen, d. h. hinteren oder der Injektionsnadel entgegengesetzten Ende einen Flansch, der auch als Fingerflansch bezeichnet werden kann, aufweisen, Eine derartig ausgebildete Spritze ist als Standartspritze erhältlich, so dass für den Autoinjektor nicht zwingend eine speziell angepasste Spritze entwickelt werden braucht. Der Kolben liegt dicht an dem Innendurchmesser des Spritzenkörpers an.

Das Gehäuse ist vorzugsweise länglich und bildet die Längsachse des Autoinjektors. Das Gehäuse ist vorzugsweise hülsenförmig und oder zylindrisch. Beispielsweise kann der Behälter verschiebbar in dem Gehäuse angeordnet sein, d. h. für ein automatisches Einstechen relativ zu dem Gehäuse in distale Richtung verschiebbar sein, so dass die Nadelspitze aus einer Öffnung am distalen Ende des Autoinjektors hervortritt und automatisch in den Patienten eingestochen werden kann. Optional kann bei einer solchen Vorrichtung die Nadelspitze nach erfolgter Produktausschüttung in das distale Ende der Vorrichtung hinein bewegt werden, insbesondere der Produktbehälter relativ zu dem Gehäuse in die proximale Richtung bewegt werden.

In bevorzugten Ausführungen ist der Produktbehälter entlang der Längsachse unverschiebbar in dem Gehäuse aufgenommen, insbesondere mittels eines Produktbehälterhalters oder Spritzenhalters, der den Produktbehälter axialfest hält und axialfest mit dem Gehäuse verbunden, insbesondere verrastet ist. Bevorzugt steht die Nadelspitze über das distale Ende des Gehäuses in distale Richtung vor. Hierdurch kann die Nadel mittels einer Bewegung des Gehäuses zum Patienten hin in die Einstichstelle eingestochen werden. Vorzugsweise ist eine Nadelschutzhülse vorgesehen, welche das distale Ende des Autoinjektors bildet und eine Öffnung für die Injektionsnadel aufweist, wobei die Nadel durch die Öffnung hindurchtreten kann. Die Nadelschutzhülse kann in ihrer Ausgangsposition in Bezug auf die Nadelspitze so angeordnet sein, dass die Nadelschutzhülse distal über die Nadelspitze steht oder dass die Nadelspitze distal über das distale Ende der Nadelschutzhülse steht. Die Nadelschutzhülse ist relativ zu dem Gehäuse aus ihrer Ausgangsposition in die proximale Richtung um einen Betätigungshub in eine betätigte Position verschiebbar, insbesondere in das Gehäuse verschiebbar, so dass die Nadel aus dem distalen Ende bzw. durch die Öffnung der Nadelschutzhülse hervortritt oder weiter hervortritt. Bevorzugt kann die Nadelschutzhülse um einen Nadelschutzhub aus der betätigten Position relativ zu dem Gehäuse in die distale Richtung in eine Nadelschutzposition verschoben werden, in der das distale Ende der Nadelschutzhülse distal über die Nadelspitze steht, um zu verhindern, dass nach erfolgter Verwendung der Vorrichtung bzw. nach erfolgter Produktausschüttung eine Verletzungsgefahr, die von einer freiliegenden Nadelspitze ausgehen würde, verringert wird, Die Nadelschutzhülse kann z. B. gegen die Kraft einer Feder, die als Nadelschutzfeder bezeichnet werden kann, in die proximale Richtung verschoben werden, wobei die Feder, die z. B. die weiter unten beschriebene zweite Feder oder eine hiervon separate Feder ist, die Nadelschutzhülse aus der betätigten Position in die distale Richtung, d. h. in die Nadelschutzposition verschieben kann. Der Autoinjektor kann ein z. B. federnd angeordnetes Verriegelungsglied aufweisen, welches die Nadelschutzhülse in ihrer Nadelschutzposition insbesondere in Bezug auf das Gehäuse verriegelt und ein Zurückschieben der Nadelschutzhülse in die proximale Richtung oder in das Gehäuse blockiert. Das Verriegelungsglied verriegelt die Nadelschutzhülse zumindest so, dass die Nadel nicht aus dem distalen Ende der Nadelschutzhülse hervortreten kann. Die Nadelschutzhülse kann z. B. aus der Nadelschutzposition nur soweit in die proximale Richtung verschoben werden, dass die Nadelspitze nicht aus dem distalen Ende der Nadelschutzhülse hervortritt.

### Kolbenstange

Der Autoinjektor umfasst ferner ein Vortriebsglied, das zumindest während der Produktausschüttung auf den Kolben wirkt, insbesondere an dem Kolben anliegt, und eine erste Feder, die direkt oder bevorzugt indirekt auf das Vortriebsglied wirkt. Das Vortriebsglied kann z. B. hülsenförmig sein. Die Feder kann vor dem Gebrauch gespannt werden z.B. als ein Zwischenschritt oder während einer Dosiseinstellung. Bevorzugt kann bei Auslieferung des Autoinjektors die Feder bereits mit so viel Energie vorgespannt sein, dass die Energie für mehrere Ausschüttungen der Produktdosis ausreicht, insbesondere für die Ausschüttung des gesamten aus dem Produktbehälter ausschüttbaren Produkts ausreicht.

### Rotationsglied und Feder

Der Autoinjektor kann ferner ein Rotationsglied umfassen, dessen Drehung bewirkt, dass die Federenergie an das Vortriebsglied abgegeben wird wodurch das Vortriebsglied in distale Richtung bewegt wird.

Das Rotationsglied kann mit der Feder insbesondere einer Torsionsfeder oder Drehfeder verbunden sein, die die zur Produktausschüttung notwendige Energie speichert und bei Bedarf abgibt. Grundsätzlich kann die Feder wendelförmig oder vorzugsweise spiralförmig sein. Die Feder kann aus einem Draht oder vorzugsweise aus einem bandförmigen Material, insbesondere Federstahl gewickelt sein. Spiralförmig gewickelte Federn werden auch als Uhrenfedern oder Triebfedern bezeichnet.

Das Drehmoment der Feder muss zwischen dem Rotationsglied und dem Vortriebsglied wirken. Die Feder kann mit dem Rotationsglied oder direkt mit dem Vortriebsglied und /oder mit einem beliebigen Element, welches axial und / oder rotativ fest in dem Gehäuse angebracht ist verbunden sein, oder mit einem Element welches axial und /oder rotativ gegenüber Gehäuse verschiebbar ist, verbunden sein. Die Feder kann axial fest in dem Gehäuse angebracht sein, sie kann aber auch axial verschiebbar zum Gehäuse sein.

Z.B. kann das Rotationsglied mit einem Ende an die Feder gekoppelt sein, wobei das andere Ende der Feder mit einem anderen Element, welches gegenüber dem Gehäuse insbesondere axial oder rotativ beweglich ist, verbunden sein kann. In einer bevorzugten Ausführungsform ist das andere Ende der Feder mit dem Gehäuse oder einem gehäusefestem Element verbunden.

Das Rotationsglied und / oder das Vortriebsglied und /oder ein anderes Element, welches mit dem Vortriebsglied gekoppelt ist, kann ein Gewinde mit einer variablen Steigung aufweisen, wobei in einem ersten Bereich das Gewinde eine grosse Steigung aufweisen kann und in weiteren Bereichen unterschiedlich grosse Steigungen möglich sind.

Aus Toleranzgründen kann im Auslieferungszustand des Autoinjektors ein Abstand zwischen Kolbenstange und Kolben bestehen. Bei der Herstellung wird versucht, den Abstand so klein wie möglich zu halten, damit der Aufprall der Kolbenstange auf den Kolben nicht zu einem Glasbruch führt. Dieser Abstand zwischen Kolbenstange und Kolben wird auch Beschleunigungsweg genannt. Um die Beschleunigung der Kolbenstange im Beschleunigungsweg zu kontrollieren bzw. abzubremsen, und Glasbruchrisiko zu minimieren, wird für den Anfang der Kolbenstangenbewegung ein Gewindeeinlaufweg mit einer grossen Steigung insbesondere auf dem Rotationsglied und / oder Vortriebsglied gewählt. Bevorzugt ist der Axialabschnitt des Gewindeeinlaufwegs grösser als der Beschleunigungsweg. Des Weiteren können in einer Lagerposition die Axialkräfte, welche insbesondere durch die Gewindeübersetzung aus dem Drehmoment der Feder entstehen durch eine grosse Steigung gering gehalten werden.

Das Gewinde bzw. die Gewindesteigung kann über die Länge des Rotationsglieds und / oder des Vortriebsglieds und / oder des Gehäuses variieren. Das Gewinde kann ein- oder mehrgängig sein. Vorzugsweise ist das Gewinde zweigängig. Die Steigung kann progressiv oder degressiv sein. Z.B. kann ein weiterer Bereich des Rotationsglieds eine kleinere Steigung aufweisen als der erste Bereich, wobei die grösste Gewindesteigung vorzugsweise nicht selbsthemmend sein kann.

Mit solch einem variierenden Gewinde ist es möglich den Abfall des Federkraftmoments zu kompensieren und während der Ausschüttung die Ausschüttkraft in einem konstanten Bereich zu halten. Es ist möglich, dass am Ende der Ausschüttbewegung eine kleine Gewindesteigung gewählt wird und somit die Ausschüttkraft erhöht wird, damit z. B. eine Stopfenreibungskraft welche am Ende der Ausschüttung zunehmen kann, kompensiert wird und eine vollständige Ausschüttung gewährleistet werden kann. Das Rotationsglied und / oder das Vortriebsglied und / oder das Gehäuse kann mehrere Bereiche mit verschiedenen Gewindesteigungen aufweisen. Z.B. kann das Gewinde eine grosse Gewindesteigung für den Gewindeeinlauf aufweisen und danach einen Bereich mit einer immer kleiner werdenden Gewindesteigung -für eine langsame Ausschüttung- und in einem Bereich mit einer kleinen Gewindesteigung -um ein vollständiges Ausschütten zu gewährleisten- enden. Selbstverständlich ist es auch möglich, dass die Gewindesteigung nach dem Gewindeeinlauf von einer kleinen Steigung in eine grosse Steigung verläuft, um am Anfang der Ausschüttung hohe Ausschüttkraft und am Ende der Ausschüttung eine kleine Ausschüttkraft zu erhalten.

Die Drehung des Rotationsglieds und / oder des Vortriebsglieds um einen bestimmten Drehwinkel bewirkt den axialen Vorschub des Vortriebsglieds um einen entsprechenden Ausschütthub. Variable Steigungswinkel ergeben variablen axialen Vorschub des Vortriebsglieds bei gleichem Drehwinkel. Durch eine Freigabe des Vortriebsglieds kann der Feder erlaubt werden, dass sie das Vortriebsglied in distale Richtung bewegen kann. Insbesondere kann das Rotationsglied so mit dem Vortriebsglied gekoppelt sein, dass es bei der Auslösung bzw. Freigabe des Vortriebsglieds für eine Produktausschüttung für eine Drehung relativ zu dem Gehäuse freigegeben ist und im nicht ausgelösten Zustand des Vortriebsglieds für eine Rotation relativ zu dem Gehäuse gesperrt ist.

Vorteilhaft kann das Rotationsglied in einem Eingriff, insbesondere einem Gewindeeingriff mit dem Vortriebsglied sein, wobei der Gewindeeingriff bzw. die Gewindemutter am Vortriebsglied oder am Rotationsglied ein oder mehrere Gewindesegmente haben kann, vorzugsweise können es zwei Gewindesegmente sein. Bevorzugt ist das Gewindesegment so gewählt, dass ein Gewinde mit einer variablen Steigung hemmungsfrei durch die Gewindesegmente gedreht werden kann. Z. B. kann das Gewindesegment in einer abgewickelten Darstellung eine Kreisform oder eine ovale Form aufweisen, insbesondere kann der Umfang zu einer Längsachse hin, verschiedene Winkel aufweisen.

Bevorzugt kann eine Flanke des Gewindesegments des Vortriebsglieds oder des Rotationsglieds verschiedene Steigungswinkel aufweisen.

Vorzugsweise wird das Gewindesegment mit insbesondere Strichberührungen an dem Gewinde des Rotationsglieds geschraubt, wobei immer ein anderer Bereich des Gewindesegments das Gewinde berührt. Insbesondere kann bei einer Schraubbewegung zwischen der Gewindestange oder dem Vortriebsglied und dem mindestens einem Gewindesegment des Vortriebsglieds bzw. der Gewindestange, die Flanke des mindestens einem Gewindesegments an dem Gewinde mit der variablen Steigung der Gewindestange oder des Vortriebsgtieds geschraubt werden, wobei unterschiedliche Bereiche der Flanke das Gewinde mit der variablen Steigung berühren. So ist bei der Schraubbewegung zwischen der Gewindestange und dem Vortriebsglied der Bereich der Gewindestange, welcher die grösste Gewindesteigung aufweist, mit dem Bereich des Gewindesegments, welcher den grössten Steigungswinkel aufweist in Berührung oder der Bereich der Gewindestange weicher die kleinste Gewindesteigung aufweist, mit dem Bereich des Gewindesegments welcher den kleinsten Steigungswinkel aufweist, in Berührung.

In einem alternativen Beispiel können das Rotationsglied eine Gewindemutter und das Vortriebsglied eine Gewindestange aufweisen oder sein, wobei das Gewinde der Gewindemutter in das Gewinde der Gewindestange eingreift und insbesondere nicht selbsthemmend ist.

Vorzugsweise ist das Rotationsglied axial fest in Bezug auf das Gehäuse oder kann sich zumindest in eine, vorzugsweise in distaler Richtung axialfest an dem Gehäuse oder einem gehäusefesten Element, wie z. B. dem Mechanikhalter abstützen, es ist auch möglich, dass das Rotationsglied einen Axialhub gegenüber dem Gehäuse ausführen kann. Insbesondere kann das Rotationsglied axial verschiebbar sein, wenn das Element an dem sich das Rotationsglied abstützt auch eine axiale Bewegung ausübt oder das Rotationsglied eine Gewindeverbindung zu einem gehäusefesten Element oder zu einem zum Gehäuse axial verschiebbaren Element aufweist,

Es ist auch möglich, dass mehrere Steuerkurven bzw. Gewinde zwischen verschiedenen Elementen wirken. Die verschiedenen Gewinde können ergänzend wirken, das heisst, für den axialen Weg addieren sich die Gewindesteigungen zueinander oder sie subtrahieren sich voneinander, somit erreicht man eine Übersetzung oder eine Untersetzung. Beispielsweise kann das Rotationsglied eine Gewindeverbindung zum Vortriebsglied aufweisen und das Vortriebsglied eine Gewindeverbindung zum Gehäuse oder zu einem gehäusefesten Element, wobei das Vortriebglied auch eine Rotationsbewegung ausführen kann, Es ist auch möglich, dass das Rotationsglied Gewindeverbindungen zum Vortriebsglied und zu einem gehäusefesten Element aufweist, wobei diese Gewindeabschnitte sich voneinander unterscheiden können und jeweils entweder variabel oder konstante Steigungsbereiche aufweisen können.

Die Gewinde können jeweils innen oder aussen an dem Rotationsglied und / oder an dem Vortriebsglied angebracht sein. Es können alle Gewindeverläufe an den verschiedenen Elementen variabel oder nur einer davon variabel sein. Z.B. kann das Rotationsglied ein stetig variables und / oder ein unstetig variables Gewinde und das Vortriebsglied eine stetige Gewindesteigung und / oder eine unstetig variables Gewindesteigung aufweisen.

Vorzugsweise kann das Gewinde mit der variablen Steigung, zumindest einen Bereich mit einer stetigen Steigungsvariation aufweisen oder/ und die Steigungsvariation des Gewindes mit der variablen Steigung zumindest bereichsweise unstetig verlaufen.

Bevorzugt können alle Elemente Gewindesegmente aufweisen und so gewählt sein, dass ein variables Gewinde hemmungsfrei durch die Gewindesegmente gedreht werden kann.

Die Gewinde, bzw. die Gewindesteigungen können variabel also progressiv oder degressiv verlaufen. Es ist auch möglich, dass die Gewinde sprunghafte Steigungen, also Bereiche mit unterschiedlicher Steigung aufweisen. Selbstverständlich kann das Gewinde mit der variablen Steigung, Bereiche mit einer stetigen Steigungsvariation aufweisen oder/ und die Variation des Gewindes mit der variablen Steigung verläuft zumindest bereichsweise unstetig.

In einer bevorzugten Ausführungsform ist das Vortriebsglied rotativ fest in Bezug auf das Gehäuse.

Durch die Verschiebung des Vortriebsglieds um den Ausschütthub wird auch der Kolben verschoben. Sofern im Auslieferungszustand zwischen dem Kolben und dem Vortriebsglied ein Abstand besteht, ist der Ausschütthub des Kolbens kleiner als der Ausschütthub des Vortriebsglieds, was bevorzugt ist, da der Kolben so bis zur Verwendung unbelastet bleibt, wodurch eine ungewollte vorzeitige Produktausschüttung vermieden wird. Grundsätzlich ist es aber auch möglich, dass das Vortriebsglied im Auslieferungszustand und nicht erst bei der Produktausschüttung an dem Kolben anliegt. Sofern im Auslieferungszustand das Vortriebsglied an dem Kolben bereits anliegt, entspricht der Ausschütthub des Kolbens dem Ausschütthub des Vortriebsglieds.

### NSH

In Ausführungsformen mit einer Nadelschutzhülse ist es bevorzugt, dass die Nadelschutzhülse auf die zweite Feder wirkt, wobei die Nadelschutzhülse für die Auslösung der Produktausschüttung aus ihrer Ausgangsposition relativ zu dem Gehäuse und entlang der Längsachse des Autoinjektors in die proximale Richtung, d. h. entgegen die Ausschüttrichtung verschiebbar ist, insbesondere um den Betätigungshub. Hierdurch wird die zweite Feder gespannt und vorzugsweise auch die Produktausschüttung, insbesondere die Bewegung des Vortriebsglieds in die Ausschüttrichtung, freigegeben bzw. ausgelöst, Die Nadelschutzhülse wird vorzugsweise dadurch aus ihrer Ausgangsposition um den Betätigungshub in ihre betätigte Position bewegt, dass ihr distales Ende an die Einstichstelle des Patienten angedrückt wird, wobei das Gehäuse relativ zu der Nadelschutzhülse in Richtung Einstichstelle verschoben wird, so dass die Nadelschutzhülse den Betätigungshub relativ zu dem Gehäuse ausführt. Hierbei wird auch die aus dem distalen Ende der Nadelschutzhülse hervortretende Nadel in die Einstichstelle eingestochen. Nach der erfolgten Produktausschüttung, insbesondere nach z. B. einer kurzen Wartezeit, wie z. B. 3 bis 10 Sekunden, nach dem mittels eines Endsignalglieds ein Signal erzeugt wurde, wird der Autoinjektor von der Einstichstelle abgenommen, wodurch die Nadelschutzhülse relativ zu dem Gehäuse aus ihrer betätigten Position um den Nadelschutzhub in die Nadelschutzposition verschoben wird, insbesondere mittels der in der zweiten Feder gespeicherten Federenergie. Durch das Abnehmen des Autoinjektors von der Einstichstelle wird auch die Nadel aus der Einstichstelle gezogen.

In bestimmten Ausführungsformen kann kinematisch zwischen der zweiten Feder und der Nadelschutzhülse ein Schaltmodul angeordnet sein, wobei das Schaltmodul von der Nadelschutzhülse in die proximale Richtung mitgenommen wird, wenn die Nadelschutzhülse aus ihrer Ausgangsposition in die proximale Richtung oder in die betätigte Position verschoben wird, und die Nadelschutzhülse in die distale Richtung verschiebt, wenn die auf das Schaltmodul wirkende Feder das Schaltmodul in die distale Richtung verschiebt. Das Schaltmodul oder ein Teil davon, wie z. B. eine Schalthülse, kann mit der Nadelschutzhülse einteilig sein oder z. B. formschlüssig verbunden, wie z. B. verschnappt, sein oder lose an der Nadelschutzhülse anliegen. Das Schaltmodul kann ein einziges Teil sein oder mehrere Teile umfassen, wobei ein mehrteiliges Schaltmodul zumindest die Schalthülse und eine Sperrhülse aufweisen kann. Die Sperrhülse kann relativ zu der Nadelschutzhülse und/oder Schalthülse z. B. entlang der Längsachse verschiebbar sein. Beispielsweise können sich die Feder an der Schalthülse und die Schalthülse an der Nadelschutzhülse abstützen. Zwischen der Sperrhülse und der Schalthülse kann ein z. B. unidirektional wirkendes Verriegelungsglied vorgesehen sein, das z. B. von der Sperrhülse gebildet wird und in die Schalthülse, insbesondere in eine Ausnehmung oder in das distale Ende, eingreift. Das Verriegelungsglied ist vorzugsweise so ausgestaltet, dass die Schalthülse während ihrer Bewegung relativ zu dem Gehäuse in die proximale Richtung die Sperrhülse über das Verriegelungsglied mitnimmt, insbesondere während der Bewegung der Nadelschutzhülse aus ihrer Ausgangsposition in die betätigte Position, und während ihrer Bewegung relativ zu dem Gehäuse in die proximale Richtung relativ zu der Sperrhülse in eine Sperrposition verschoben wird, insbesondere während der Verschiebung der Nadelschutzhülse aus ihrer betätigten Position in die Nadelschutzposition, wobei in der Sperrposition das oder ein anderes Verriegelungsglied, wie z. B. das weiter oben genannte, eine Bewegung der Schalthülse relativ zu der Sperrhülse in die proximale Richtung sperrt. Hierdurch wird vorteilhaft verhindert, dass die Nadelschutzhülse aus ihrer Nadelschutzposition für eine erneute Freigabe der Nadelspitze in das Gehäuse zurückgeschoben werden kann,

Beispielsweise kann die Schalthülse eine erste Ausnehmung aufweisen, in welche das Verriegelungsglied der Sperrhülse lösbar eingreift, wenn die Nadelschutzhülse aus ihrer Ausgangsposition in ihre betätigte Position verschoben wird. Z. B. kann die Schalthülse eine zweite Ausnehmung aufweisen, in welche das Verriegelungsglied oder ggf. das andere Verriegelungsglied eingreift, wenn die Nadelschutzhülse in ihrer Nadelschutzposition ist. Die erste und zweite Ausnehmung können vorzugsweise mit einem Abstand, der in etwa dem Nadelschutzhub entspricht, zueinander entlang der Längsachse angeordnet sein. Selbstverständlich ist auch eine Umkehr der Anordnung der Ausnehmungen und des Verriegelungsglieds oder der Verriegelungsglieder möglich, d. h., dass das zumindest eine Verriegelungsglied an der Schalthülse und die mindestens eine Ausnehmung, d. h. die erste Ausnehmung und ggf. die zweite Ausnehmung an der Sperrhülse gebildet sein können.

Das Verriegelungsglied und ggf, das andere Verriegelungsglied können federnd, insbesondere jeweils an einem federnden Arm, angeordnet sein. Vorzugsweise kann die Schalthülse die Sperrhülse umgeben und/oder führen.

### Kappe

Weiterhin kann der Autoinjektor eine Verschlusskappe bzw. ein proximales Gehäuseteil aufweisen, welches am proximalen Ende des Gehäuses anbringbar ist und das proximale Ende des Autoinjektors bildet. Bevorzugt kann die Kappe oder das proximale Gehäuseteil Raum für die Antriebsfeder, bzw. erste Feder insbesondere Spiralfeder schaffen. Die Verschlusskappe oder das proximale Gehäuseteil kann mit dem Gehäuse so gekoppelt sein, dass sie sich gegenüber verdrehen lässt. Z.B. kann das proximale Gehäuseteil ein Dosierknopf oder ein Ladeknopf sein, wodurch die Feder vor der Injektion gespannt werden kann. In einer bevorzugten Ausführungsform kann die Verschlusskappe oder das proximale Gehäuseteil mit dem Gehäuse formschlüssig, alternativ kraft- oder stoffschlüssig verbunden sein. Besonders bevorzugt kann die Kappe oder das zweite Gehäuseteil lösbare und unlösbare Verbindungen aufweisen. Bevorzugt kann die Kappe oder das proximale Gehäuseteil in einem ersten Montageschritt lösbar mit dem Gehäuse verbunden werden und in einem zweiten Montageschritt zum Einfügen des Produktbehälters von dem Gehäuse gelöst und nach dem Einfügen des Spritzenkörpers unlösbar miteinander verbunden werden. Vorzugsweise wird die Kappe oder das proximale Gehäuseteil in dem zweiten Montageschritt unlösbar mit dem Gehäuse verrastet. Eine separate Kappe bzw. ein proximales Gehäuseteil mit lösbaren und unlösbaren Verrastungen hat den Vorteil, dass die Montage der Vorrichtung erleichtert wird, wobei für die Endmontage zumindest ein Teil der Bauteile über das proximale Ende des Gehäuses eingebaut werden und mit der Kappe in der Vormontage lösbar fixiert werden. Nach vorübergehender Entfernung der Kappe bzw. des proximalen Gehäuseteils in der Endmontage kann der Spritzenkörper über das proximale Ende des Gehäuses eingesetzt und die beiden Gehäuseteile unlösbar miteinander formschlüssig verrastet, alternativ kraft- oder stoffschlüssig zu verbunden werden.

### StartKlick

In bevorzugten Ausführungen kann der Autoinjektor ein Halteelement aufweisen, an dem sich z. B. ein Ende der zweiten Feder, insbesondere das proximale Ende der zweiten Feder, abstützt. Alternativ kann sich die Feder mit ihrem proximalen Ende an dem Gehäuse oder einem gehäusefesten Element abstützen.

Die zweite Feder kann sich mit ihrem distalen Ende z.B. an dem Gehäuse oder einem gehäusefesten Element abstützen. Besonders bevorzugt stützt sich die zweite Feder mit ihrem distalen Ende an der Nadelschutzhülse oder einem Element, welches, insbesondere bei der Verschiebung der Nadelschutzhülse relativ zu dem Gehäuse, mit der Nadelschutzhülse verschoben wird, ab. Z.B kann das Element das Schaltmodul oder insbesondere die Schalthülse sein. Das Halteelement selbst kann gehäusefest oder in Bezug auf das Gehäuse verschiebbar angeordnet sein. Das Halteelement kann ein erstes Eingriffselement aufweisen, welches vor dem Auslösen der Produktausschüttung in das Vortriebsglied eingreift, wodurch das Vortriebsglied daran gehindert wird, sich relativ zu dem Halteelement und/oder dem Gehäuse in die Ausschüttrichtung zu bewegen. Der Eingriff des ersten Eingriffselements in das Vortriebsglied ist für die Produktausschüttung lösbar, Wenn der Eingriff gelöst ist, ist das Vortriebsglied für die Bewegung in die Ausschüttrichtung freigegeben. Die erste Feder kann das Vortriebsglied relativ zu dem Halteelement und/oder dem Gehäuse um den Ausschütthub in die Ausschüttrichtung verschieben. Das Vortriebsglied kann eine Ausnehmung für das erste Eingriffselement des Halteelements aufweisen, wobei diese Kopplung zwischen dem Vortriebsglied und dem Halteelement gelöst ist, wenn das Halteelement, insbesondere das erste Eingriffselement, aus dem Eingriff mit dem Vortriebsglied, insbesondere der Ausnehmung des Vortriebsglieds ausgerückt ist. Insbesondere kann das erste Eingriffselement dadurch aus dem Eingriff mit dem Vortriebsglied gelöst werden, dass die Nadelschutzhülse aus der Ausgangsposition um den Betätigungshub in die betätigte Position verschoben wird. Beispielsweise kann das erste Eingriffselement von der Nadelschutzhülse oder dem Schaltmodul, insbesondere der Sperrhülse in den axialfesten Eingriff mit dem Vortriebsglied gehalten werden, wenn die Nadelschutzhülse nicht in ihrer betätigten Position oder in ihrer Ausgangsposition ist. Beispielsweise kann ein Innenumfang der Nadelschutzhülse oder des Schaltmoduls, insbesondere der Sperrhülse, das erste Eingriffselement in dem Eingriff mit dem Vortriebsglied halten.

Durch das Verschieben der Nadelschutzhülse in ihre betätigte Position kann die Nadelschutzhülse oder das Schaltmodul, insbesondere die Sperrhülse, dem ersten Eingriffselement erlauben, insbesondere mit einer Bewegung quer zu der Längsachse des Autoinjektors aus dem Eingriff mit dem Vortriebsglied auszurücken. Beispielsweise kann eine erste Ausnehmung, insbesondere für das zweite Eingriffselement, welche an der Nadelschutzhülse oder dem Schaltmodul, insbesondere der Sperrhülse, gebildet ist, bezogen auf die Längsachse auf der gleichen Position angeordnet sein, wie das erste und/oder zweite Eingriffselement, so dass das erste Eingriffselement aus dem Eingriff mit dem Vortriebsglied ausrücken kann. Beispielsweise kann das Vortriebsglied das erste Eingriffselement aus dem Eingriff mit dem Vortriebsglied drücken, wenn die Nadelschutzhülse in ihrer betätigten Position ist.

Das erste Eingriffselement kann z. B. radial zu der Längsachse hinweisen und/oder an einem federnden Arm des Halteelements angeordnet sein.

Das Halteelement kann - wie erwähnt - ein zweites Eingriffselement aufweisen, welches durch die Ausrückbewegung des ersten Eingriffselements aus dem Vortriebsglied in einen Eingriff mit der Nadelschutzhülse oder dem Schaltmodul, insbesondere der Sperrhülse bewegbar ist. Das zweite Eingriffselement kann z. B. an dem Arm, an dem das erste Eingriffselement angeordnet ist, angeordnet sein und/oder z. B. radial von der Längsachse weg weisen. Das erste Eingriffselement und das zweite Eingriffselement können so aufeinander abgestimmt sein, dass das zweite Eingriffselement bereits axialfest in seine Ausnehmung, welche von der Nadelschutzhülse oder dem Schaltmodul, insbesondere der Sperrhülse, gebildet wird, eingreift, wenn das erste Eingriffselement noch nicht vollständig aus dem Eingriff mit dem Vortriebsglied gelöst ist. Hierdurch wird vorteilhaft erreicht, dass zuerst die axialfeste Verbindung zwischen dem Halteelement und der Nadelschutzhülse oder dem Schaltmodul hergestellt ist, bevor die axialfeste Verbindung zwischen dem Halteelement und dem Vortriebsglied gelöst wird.

Insbesondere wenn das zweite Eingriffselement in seiner Ausnehmung ist, kann sich das Vortriebsglied relativ zu dem Halteelement in distale Richtung bewegen, insbesondere aufgrund der in der vorgespannten ersten Feder gespeicherten Energie. Das Vortriebsglied kann das zweite Eingriffselement daran hindem, aus dem axialfesten Eingriff mit der Nadelschutzhülse oder dem Schaltmodul, insbesondere der Sperrhülse auszurücken, wenn sich das Vortriebsglled relativ zu dem Halteelement in die distale Richtung bewegt.

Da die axialfeste Kopplung zwischen dem Vortriebsglied und dem Halteelement nun aufgehoben ist, ist das Halteelement von der zweiten Feder relativ zum Gehäuse nach proximal bewegbar. Insbesondere wird dabei die Sperrhülse um einen Abstand zwischen der Sperrhülse und einem Startsignalanschlag bewegbar, Die zweite Feder kann das Halteelement und/ oder die Sperrhülse auf diesem Abstand beschleunigen, wodurch die Sperrhülse mit einer Geschwindigkeit auf den Startsignalanschlag auftrifft, so dass ein Startimpuls abgegeben wird, der ein akustisches (hörbares) und/ oder taktiles (fühlbares) Signal erzeugt.

Der Startsignalanschlag kann von dem Gehäuse oder einem zumindest axialfest, vorzugsweise auch drehfest mit dem Gehäuse verbundenem Element gebildet werden. Beispielsweise kann dieses Element der Mechanikhalter sein. Bevorzugt ist der Signalanschlag entlang der Längsachse des Autoinjektors so angeordnet, dass er in einer Flucht mit der Sperrhülse angeordnet ist. Hierdurch wird erreicht, dass die Sperrhülse mit einer Bewegung des Halteelements entlang der Längsachse des Autoinjektors an dem Startsignalanschlag anschlägt.

### End of Injection EOI Signal

Erfindungsgemäß weist der Autoinjektor einen Endsignalanschlag auf. Wie bereits erwähnt kann die zweite Feder eine auf das Halteelement entgegen der Ausschüttrichtung oder in die proximale Richtung wirkende Federkraft ausüben, Insbesondere kann sich die zweite Feder z. B. mit ihrem proximalen Ende an dem Endsignal abstützen.

Die Feder kann somit bevorzugt eine Mehrfachfunktion erfüllen, da sie Kraft zum Verschieben der Nadelschutzhülse bzw. des Schaltmoduls aufbringt und Kraft auf das Halteelement, für ein Startsignal und für ein Endsignal, ausübt.

Wie bereits oben erwähnt kann das Vortriebsglied das zweite Eingriffsglied daran hindem, aus dem Eingriff mit der Nadelschutzhülse oder dem Schaltmodul auszurücken, wenn sich das Vortriebsglied relativ zu dem Halteelement in die distale Richtung bewegt. Das Vortriebsglied erlaubt dem zweiten Eingriffsglied am Ende des Ausschütthubs, dass es aus dem Eingriff mit der Nadelschutzhülse oder dem Schaltmodul bzw. der Sperrhülse ausrückt. Wenn das zweite Eingriffsglied am Ende der Ausschüttung aus dem Eingriff mit der Nadelschutzhülse oder dem Schaltmodul bzw. der Sperrhülse ausgerückt ist, wird das Halteelement von der zweiten Feder entgegen die Ausschüttrichtung beschleunigt und schlägt an dem Endsignalanschlag an.

Der Endsignalanschlag kann von dem Gehäuse oder einem zumindest axialfest, vorzugsweise auch drehfest mit dem Gehäuse verbundenem Element gebildet werden, Beispielsweise kann dieses Element der Mechanikhalter sein. Bevorzugt ist der Endsignalanschlag entlang der Längsachse des Autoinjektors so angeordnet, dass er in einer Flucht mit dem Halteelement angeordnet ist. Hierdurch wird erreicht, dass das Halteelement mit einer Bewegung entlang der Längsachse des Autoinjektors an dem Endsignalanschlag anschlägt

### Sperre der Nadelschutzhülse NSH

Insbesondere in Ausführungen, in denen die distale Ausnehmung für das zweite Eingriffselement des Halteelements von der Nadelschutzhülse oder der Schalthülse insbesondere der Sperrhülse gebildet wird, ist es bevorzugt, dass das zweite Eingriffselement am Ende des Ausschütthubs aus seiner Ausnehmung ausrückt, um die Nadelschutzhülse nach der Verabreichung des Produkts aus der betätigten Position in die Nadelschutzposition bewegen zu können. Das Vortriebsglied kann hierzu eine proximale Ausnehmung aufweisen, in welche das erste Eingriffselement einrücken kann, wobei das zweite Eingriffselement gleichzeitig aus seiner Ausnehmung ausrückt. Die distale Ausnehmungen an der Sperrhülse kann auch das distale Ende der Sperrhülse bilden. Es ist auch möglich, dass die proximale Ausnehmung an dem Vortriebsglied das proximale Ende des Vortriebsglieds darstellen kann.

In Ausführungsformen, mit einem Schaltmodul, welches eine Schalthülse und eine Sperrhülse aufweist, ist es bevorzugt, dass die Sperrhülse mit einem nach innen weisenden Sperrarm in das Gehäuse oder in ein gehäusefesten Element, wie z. B. in den Mechanikhalter eingreift und so die Sperrhülse daran hindert, relativ zu dem Gehäuse in die distale Richtung bewegt zu werden, wobei die Schalthülse und/oder die Nadelschutzhülse relativ zu der Sperrhülse, insbesondere aufgrund der in der zweiten Feder gespeicherten Energie in die distale Richtung verschiebbar sind, wodurch die Nadelschutzhülse insbesondere in ihre Nadelschutzposition bewegt wird, Wie bereits beschrieben und nur der Vollständigkeit halber angemerkt, kann das Verriegelungsglied zwischen der Sperrhülse und der Schalthülse in einen Eingriff gelangen, der verhindert, dass die Schalthülse relativ zu der Sperrhülse in die proximale Richtung verschiebbar ist. Vorzugsweise wird eine Bewegung der Sperrhülse in die proximale Richtung dadurch verhindert, dass die Sperrhülse entweder an dem Gehäuse oder einem gehäusefesten Element, wie z. B. an dem Mechanikhalter, anstößt.

### Spritzenhalter

Weiterhin weist der Autoinjektor einen Produktbehälterhalter, insbesondere einen Spritzenhalter insbesondere für einen Autoinjektor, bei dem der Produktbehälter in Bezug auf das Gehäuse unverschiebbar ist bzw, für einen Autoinjektor der oben beschriebenen Art.

Die Erfindung geht von einem Spritzenmodul aus, welches insbesondere zur Verwendung in einem Autoinjektor vorgesehen ist. Insbesondere kann ein Autoinjektor vorgesehen sein, der ein solches Spritzenmodul aufweist. Das Spritzenmodul umfasst eine Spritze und einen Spritzenhalter. Die Spritze weist einen Spritzenkörper, einen Kolben und eine Nadel auf, wobei die Nadel z. B. unlösbar an einem Nadelhalteabschnitt der Spritze befestigt ist und der Kolben in einem zylindrischen Abschnitt des Spritzenkörpers verschiebbar angeordnet ist, wobei der Spritzenkörper einen sich verjüngenden Abschnitt oder Bereich aufweist, der zwischen dem zylindrischen Abschnitt und dem Nadelhalteabschnitt angeordnet ist. Die Spritze weist ferner eine Nadelschutzkappe auf, welche z. B. ein sogenanntes soft needle shield oder vorzugsweise ein rigid needle shield RNS sein kann. Ein soft needle shield ist vorzugsweise aus einem gummielastischen Kunststoff gebildet, wobei ein rigid needle shield aus einer Hülse aus Hartkunststoff gebildet ist, in der eine Hülse aus einem gummielastischen Kunststoff angeordnet ist. Die Hülse aus gummielastischem Kunststoff und die Hülse aus Hartkunststoff bilden zusammen das rigid neelde shield. Die Nadelschutzkappe, welche die Nadel abdeckt und an dem sich insbesondere konisch in Richtung Nadelspitze erstreckenden Nadelhalteabschnitt befestigt ist, hält die Nadel vorzugsweise geschützt vor Schmutz und steril. Über dem sich verjüngenden Abschnitt zwischen dem zylindrischen Abschnitt und der Nadelschutzkappe, insbesondere der Hülse aus Hartkunststoff, ist eine Lücke gebildet.

Der Spritzenhalter weist mindestens ein Eingriffsglied, insbesondere eine Schulter, an welcher sich der verjüngende Abschnitt der Spritze in die distale Richtung abstützt und welche in die Lücke zwischen der Nadelschutzkappe und dem zylindrischen Abschnitt eingreift, auf. Vorteilhaft wird durch das Anliegen des sich verjüngenden Abschnitts an der mindestens einen Schulter verhindert, dass sich die Spritze relativ zu dem Spritzenhalter in die distale Richtung bewegen kann.

Es ist bevorzugt, dass zwischen der Schulter und der Nadelschutzkappe ein Spalt ist oder besteht, so dass die Nadelschutzkappe von der Schulter unbelastet bleibt. Hierdurch wird vorteilhaft verhindert, dass die Sterilität der Nadel durch ein unbeabsichtigtes Verschieben der Nadelschutzkappe durch die Schulter beeinträchtigt wird.

In bevorzugten Ausführungen kann der Spritzenkörper an seinem proximalen Ende einen Fingerflansch aufweisen, wobei zwischen dem Fingerflansch und dem Spritzenkörper ein Spalt gebildet ist, wenn der sich verjüngende Abschnitt an der Schulter anliegt, wodurch der Fingerflansch im Wesentlichen unbelastet bleibt. Hierdurch wird vorteilhaft verhindert, dass der Fingerflansch überbelastet wird und den Spritzenkörper zerbricht.

Es ist ferner bevorzugt, dass der Spritzenhalter mindestens ein Halteglied, insbesondere eine nach außen gerichtete Abragung aufweist, mit dem der Spritzenhalter axialfest mit einem Gehäuse des Autoinjektors verbindbar oder verbunden ist, insbesondere verschnappt oder verschnappbar ist.

In bevorzugten Ausführungen kann der Spritzenhalter mindestens einen Nocken aufweisen, der federnd, insbesondere an einem Arm angeordnet ist und z. B. distal des Halteglieds angeordnet ist. Der mindestens eine Nocken kann eine Nadelschutzhülse an der Bewegung aus ihrer Ausgangsposition in ihre betätigte Position so hemmen oder hindem, dass beim Überschreiten einer auf die Nadelschutzhülse entlang der Längsachse L des Autoinjektors ausgeübten Grenzkraft der mindestens eine Nocken aus dem Eingriff mit der Nadelschutzhülse gedrückt wird, wodurch die Nadelschutzhülse schlagartig in ihre betätigte Position relativ zu dem Gehäuse verschiebbar ist.

Das Gehäuse des Autoinjektors kann z. B. einen Halteabschnitt aufweisen, der an dem Spritzenhalter, insbesondere an einer Außenfläche oder einem Außenumfang des Spritzenhalters anliegt und das mindestens eine Eingriffsglied daran hindert, sich quer zur Längsachse von der Längsachse weg zu bewegen, Insbesondere kann der Halteabschnitt ringförmig sein und das mindestens eine Eingriffsglied, vorzugsweise zwei oder drei oder vier Eingriffsglieder umgeben, so dass das mindestens eine Eingriffsglied innerhalb des Halteabschnitts angeordnet ist. Für die Montage bzw. das Einlegen der Spritze in den Spritzenhalter befindet sich der Spritzenhalter außerhalb des Eingriffs mit dem Halteabschnitt des Gehäuses. Wenn die Spritze vollständig in den Spritzenhalter eingelegt ist, insbesondere das mindestens eine Eingriffsglied in die Lücke zwischen dem sich verjüngenden Abschnitt und der Nadelschutzkappe eingreift, wird das Spritzenmodul bzw. der Spritzenhalter in den Eingriff mit dem Halteabschnitt gebracht, so dass verhindert wird, dass sich das mindestens eine Eingriffsglied aus dem Eingriff mit dem verjüngenden Abschnitt quer zur Längsachse, insbesondere von der Längsachse weg oder nach außen, bewegt.

Die Erfindung und weitere Aspekte der Erfindung wurden anhand mehrerer bevorzugter Ausführungen beschrieben. Im Folgenden werden besonders bevorzugte Ausführungen anhand von Figuren beschrieben. Die dabei offenbarten Merkmale bilden den Gegenstand der Erfindung einzeln und in jeglicher Merkmalskombination vorteilhaft weiter. Es zeigen:
- Figur 1: eine Explosionsdarstellung eines Autoinjektors gemäß einer besonders bevorzugten Ausführungsform,
- Figuren 2a-2b: der Autoinjektor aus Figur 1 in einem Auslieferungszustand, wobei die Figuren 2a bis 2b durch die Längsachse der Vorrichtung verlaufende Schnittansichten sind, wobei die Schnittansichten um die Längsachse winkelversetzt sind,
- Figuren 3a-3b: die Vorrichtung und die Ansichten aus den Figuren 2a-2b, wobei sich eine Nadelschutzhülse in ihrer betätigten Position befindet,
- Figuren 4a-4b: die Vorrichtung und die Ansichten aus den Figuren 2a-2b, wobei ein Signal, welches den Anfang der Produktausschüttung signalisiert, erzeugt wird,
- Figuren 5a-5b: die Vorrichtung und die Ansichten aus den Figuren 2a-2b, wobei ein Vortriebsglied am Ende seines Ausschütthubs gezeigt wird,
- Figuren 6a-6b: die Vorrichtung und die Ansichten aus den Figuren 2a-2b, wobei ein Signal, welches das Ende der Produktausschüttung signalisiert, erzeugt wird,
- Figuren 7a-7b: die Vorrichtung und die Ansichten aus den Figuren 2a-2b, wobei die Nadelschutzhülse in ihrer Nadelschutzposition ist,
- Figuren 8a-8c: Darstellung des Rotationsglieds und des Vortriebsglieds und Ausführungsformen davon

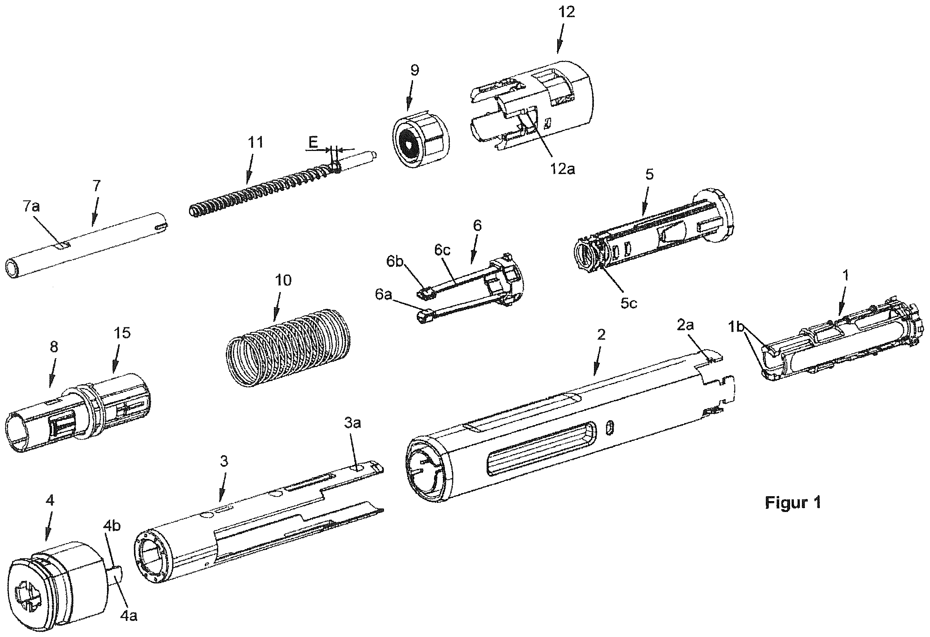

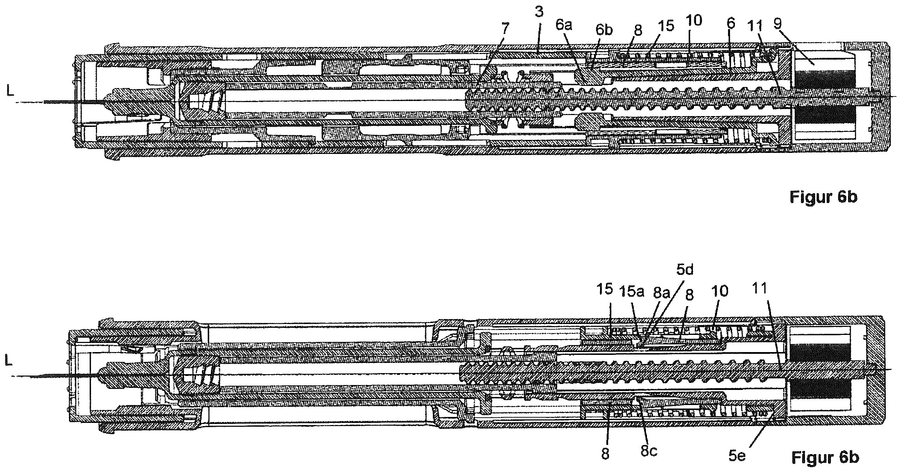

Bezugnehmend auf die Figuren 1-8c werden nun die strukturellen Merkmale und die Funktion des bevorzugten Autoinjektors beschrieben.

Der Autoinjektor weist ein hülsenförmiges, längliches Gehäuse 2 mit einer Längsachse L auf, welches an seinem proximalen Ende eine Verschlusskappe 12 aufweist, die formschlüssig mit dem Gehäuse 2 dreh- und axialfest verbunden ist und das proximale Ende des Autoinjektors bildet. Die Verschlusskappe 12 ist mit dem Gehäuse 2 verschnappt. Hierfür weist die Verschlusskappe 12 ein Rastglied 12a auf, welches in eine Ausnehmung 2a am Gehäuse 2 eingerastet ist, vorzugsweise so, dass die Verschlusskappe 12 nicht oder nicht ohne weiteres von dem Gehäuse 2 lösbar ist.

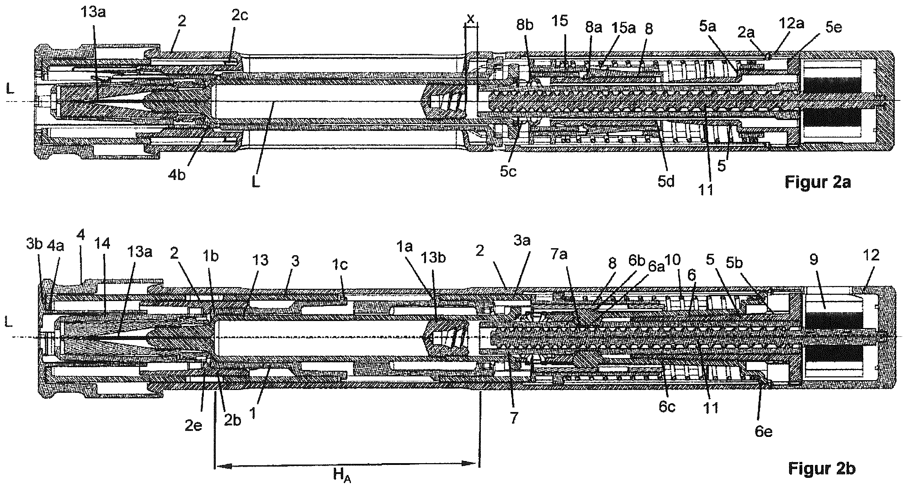

An dem distalen Ende des Autoinjektors ist in seinem Auslieferungszustand (Figuren 2a-2b) eine Abziehkappe 4 angeordnet, die vor der Verwendung des Autoinjektors abgezogen, oder abgedreht, und entfernt wird.

In dem Gehäuse 2 ist ein Produktbehälter 13 in der Gestalt einer Spritze in Bezug auf das Gehäuse 2 - abgesehen von der Montage des Autoinjektors - entlang der Längsachse L unverschiebbar aufgenommen. Der Produktbehälter 13 weist einen hülsenförmigen Spritzenkörper auf, der einen Kolben 13b, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 13b ist ein flüssiges Produkt, insbesondere Medikament, innerhalb des Spritzenkörpers angeordnet, wobei das flüssige Produkt durch Verschieben des Kolbens 13b in eine Ausschüttrichtung, d. h. in distale Richtung oder zu der Injektionsnadel 13a hin durch die hohle Injektionsnadel 13a aus dem Produktbehälter 13 ausgeschüttet wird. Der Spritzenkörper weist an seinem proximalen Ende einen sogenannten Fingerflansch auf, der radial nach außen über den Außenumfang des zylindrischen Spritzenkörpers ragt.

Der Produktbehälter 13 ist in einem Produktbehälterhalter, der als Spritzenhalter 1 bezeichnet wird, so aufgenommen, dass er zumindest gegen eine Bewegung entlang der Längsachse L in distale Richtung relativ zu dem Spritzenhalter 1 gesichert ist. Der Spritzenhalter 1 ist, wie am besten aus Figur 2a ersichtlich ist, mit dem Gehäuse 2 formschlüssig verbunden, insbesondere verrastet. Das Gehäuse 2 weist hierzu Ausnehmungen auf, in die Rastelemente, die hier am proximalen Ende des Spritzenhalters 1 gebildet werden, eingreifen. Der Spritzenhalter 1 weist mindestens eine nach innen ragende Schulter 1b auf, an der sich ein verjüngender Abschnitt des Produktbehälters 13, der distal des zylindrischen den Kolben 13b führenden Spritzenkörperabschnitts, angebracht ist, abstützt.

Um zu verhindern, dass der Produktbehälter 13 relativ zu dem Spritzenhalter 1 in die proximale Richtung verschiebbar ist, wird der Produktbehälter 13 an seinem proximalen Ende durch einen auf den Spritzenkörper wirkenden Halter in den Eingriff mit der Schulter 1b gedrückt, Der Halter wird von einem Haltefederabschnitt 5c eines Mechanikhalters 5 gebildet. Der Mechanikhalter 5 ist in Bezug auf das Gehäuse 2 entlang der Längsachse L insbesondere unverschiebbar und/oder drehfest angeordnet. Der hülsenförmige Mechanikhalter 5 kann mit dem Gehäuse 2 verschnappt sein. Durch den Haltefederabschnitt 5c können Längenunterschiede des Produktbehälters 13, die aufgrund von Herstellungstoleranzen entstehen können, ausgeglichen werden, wobei der feste Sitz des Produktbehälters 13 an der Schulter 1b sichergestellt ist.

Der Produktbehälter 13 ist in Bezug auf das Gehäuse 2 so angeordnet, dass die Nadelspitze distal über das distale Ende des Gehäuses 2 übersteht. Im Ausgangs- oder Auslieferungszustand des Autoinjektors, d. h., wenn die Abziehkappe 4 an dem Autoinjektor angeordnet ist, wird die Nadel 13a von einer Nadelabdeckkappe 14, die in dem gezeigten Beispiel als sogenanntes und dem Fachmann bekanntes rigid needle shield, alternativ als soft needle shield, ausgebildet ist, abgedeckt, um die Nadel 13a vor Verschmutzung zu schützen bzw. die Nadel 13a und das Medikament steril zu halten. Das rigid needle shield 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter 1b ist zwischen dem Spritzenkörper und dem proximalen Ende des rigid needle shield 14 angeordnet, insbesondere so, dass zwischen dem rigid needle shield 14 und der Schulter 1b ein - wenngleich auch geringer - Spalt entsteht, um zu verhindern, dass die Schulter 1b eine Kraft auf das rigid needle shield 14 ausübt, wodurch z. B. die Sterilität der Nadel 13a oder des flüssigen Produkts gefährdet werden könnte. Die Abziehkappe 4 ist mit dem Gehäuse 2 oder einer Nadelschutzhülse 3 lösbar verschnappt, wobei diese Verschnappung gelöst wird, wenn die Abziehkappe 4 von dem Gehäuse 2 oder der Nadelschutzhülse 3 entfernt wird. Die Verschnappung wird in dem gezeigten Beispiel durch eine Schnappgeometrie 3b der Nadelschutzhülse 3 und einem Schnapphaken 4a der Abziehkappe 4 gebildet (Figur 2b). Diese Schnapphaken 4a sichern die Abziehkappe 4 weiter gegen eine proximale Bewegung relativ zum Gehäuse 2 indem sie auf dem Gehäuse 2 oder an einer distalen Stirnseite an dem Spritzenhalter 1 eine gehäusefeste Abstützung finden. Die Abziehkappe 4 weist ferner insbesondere an einem Schnapphaken 4a mindestens einen Schnapper 4b auf, der in eine Lücke zwischen dem Spritzenkörper, insbesondere dessen sich verjüngenden Bereichs, und dem proximalen Ende des rigid needle shield 14 eingreift. Wenn die Abziehkappe 4 von dem Autoinjektor entfernt wird, hakt der Schnapper 4b in das proximale Ende des rigid needle shield 14 ein, wodurch das rigid needle shield 14 von dem Produktbehälter 13 gelöst und zusammen mit der Abdeckkappe 4 von dem Autoinjektor entfernt wird.

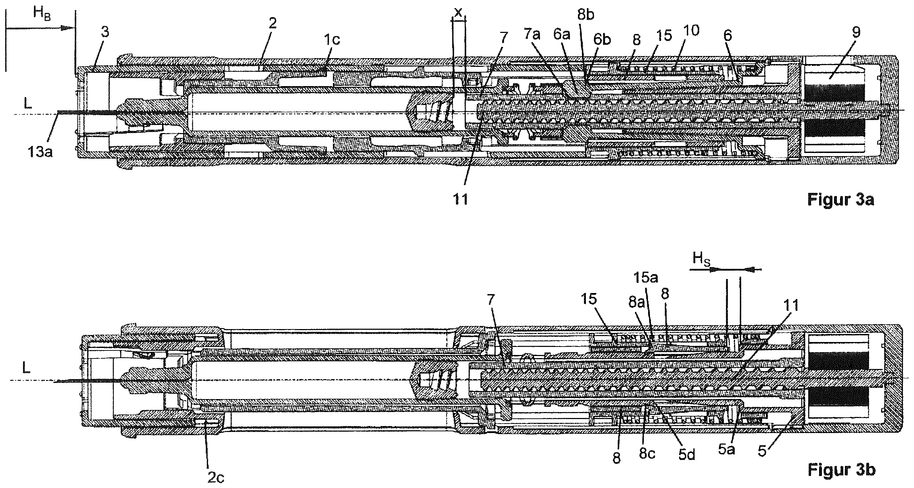

Der Autoinjektor weist eine Nadelschutzhülse 3 auf, die relativ zu dem Gehäuse 2 und entlang der Längsachse L um einen Betätigungshub H_{B} (Fig. 3a, 3b) in die proximale Richtung in eine betätigte Position verschiebbar ist, um eine Produktausschüttung auszulösen. In der Ausgangsposition der Nadelschutzhülse 3, wie sie in den Figuren 2a-2b gezeigt wird, wobei die Abziehkappe 4 abgenommen ist, steht das distale Ende der Nadelschutzhülse 3 distal über die Nadelspitze der Nadel 13a über, so dass ein Zugriff auf die Nadelspitze zunächst verhindert wird. Durch Verschieben der Nadelschutzhülse 3 um den Betätigungshub H_{B} wird die Nadelschutzhülse 3 soweit in die proximale Richtung verschoben, dass die Nadel 13a aus dem distalen Ende der Nadelschutzhülse 3 hervortritt, insbesondere mit einer Länge hervortritt, welche der Injektionstiefe der Nadel in die Einstichstelle entspricht. Bevorzugt soll die Nadel 13a soweit über das distale Ende der Nadelschutzhülse 3 überstehen, dass eine subkutane oder intramuskuläre Injektion erfolgen kann. Insbesondere kann das Gehäuse 2 einen Anschlag 2c (Fig. 3b) bilden, an dem die Nadelschutzhülse 3 in der betätigten Position anliegt.

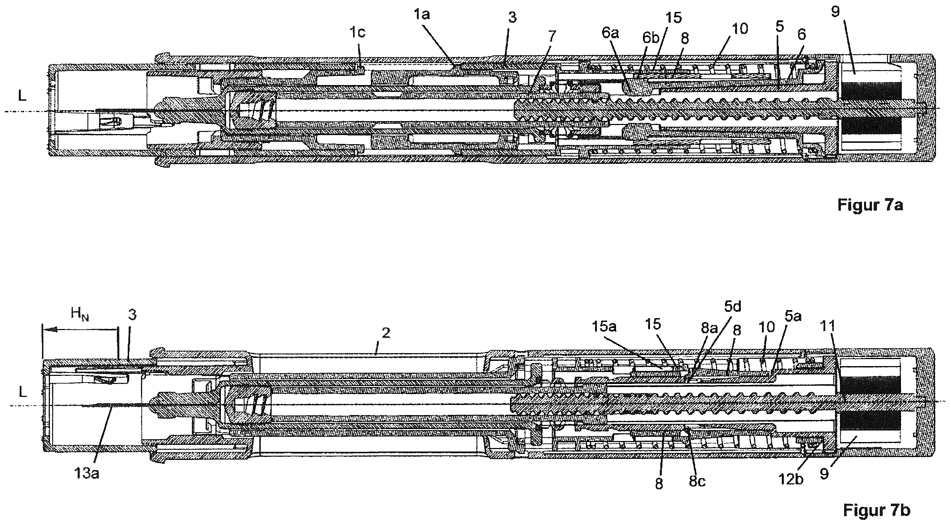

Nach der erfolgten Injektion kann die Nadelschutzhülse 3 relativ zu dem Gehäuse 2 aus der betätigten Position entlang der Längsachse L um einen Nadelschutzhub H_{N} in die distale Richtung in eine Nadelschutzposition (Figuren 7a-7b) verschoben werden, In der Nadelschutzposition steht das distale Ende der Nadelschutzhülse 3 distal über die Nadelspitze über, so dass ein Zugriff auf die Nadelspitze verhindert und eine Verletzungsgefahr verringert wird. Die Nadelschutzhülse 3 kann, wie weiter unten beschrieben wird, gegen erneutes Zurückschieben aus der Nadelschutzposition blockiert werden.

Der Spritzenhalter 1 weist eine Abragung 1a, die radial nach außen ausgebildet ist, auf, wobei die Abragung 1a in eine schlitzförmige Ausnehmung der Nadelschutzhülse 3, die zwischen dem Gehäuse 2 und dem Spritzenhalter 1 angeordnet ist, eingreift. In der Ausgangsposition der Nadelschutzhülse 3 (Figuren 2a-2b) und/oder in der Nadelschutzposition der Nadelschutzhülse 3 (Figuren 7a-7b) liegt die Nadelschutzhülse 3, insbesondere das proximale Ende der schlitzförmigen Ausnehmung an der Abragung 1a an, wodurch eine Bewegung der Nadelschutzhülse 3 in die distale Richtung verhindert wird. In diese schlitzförmige Ausnehmung, alternativ in eine andere Ausnehmung der Nadelschutzhülse 3, kann ein Nocken 1c, der federnd an dem Spritzenhalter 1 angeordnet und von dem Spritzenhalter 1 gebildet ist, eingreifen. Der Nocken 1c ist so gestaltet, dass bei dem Versuch, die Nadelschutzhülse 3 aus der Ausgangsposition in die betätigte Position zu verschieben, der Nocken 1c die Verschiebung der Nadelschutzhülse 3 zunächst verhindert, wobei der Nocken 1c herausgedrückt wird, wenn die auf die Nadelschutzhülse 3 zum Zurückschieben aufgebrachte Kraft einen gewissen Schwellwert überschreitet, wodurch die Nadelschutzhülse 3 schlagartig in die betätigte Position zurückgeschoben wird. Die Nadel 13a kann hierdurch schlagartig in die Einstichstelle eingestochen werden. Zum Einstechen der Nadel 13a bzw. zum Verschieben der Nadelschutzhülse 3 in die betätigte Position wird das distale Ende der Nadelschutzhülse 3 auf die Einstichstelle angesetzt, wobei das Gehäuse 2 dann in Richtung Einstichstelle gedrückt wird, wobei wenn die Andrückkraft den oben genannten Schwellwert überschreitet, das Gehäuse 2 schlagartig zu der Einstichstelle hin und die Nadelschutzhülse 3 relativ zu dem Gehäuse 2 in die betätigte Position verschoben wird.

Das Gehäuse 2 weist einen ringförmigen Halteabschnitt oder Ringabschnitt 2b auf, der insbesondere das distale Ende des Spritzenhalters 1 insbesondere ringförmig umgibt, und daran anliegt, wodurch die mindestens eine Schulter 1b in dem Eingriff mit dem verjüngenden Abschnitt des Spritzenkörpers gehalten wird. Ferner weist das Gehäuse 2 im Bereich des Halteabschnitts 2b einen Translationsanschlag in der Gestalt einer Halteschulter 2e auf, die verhindert, dass der Spritzenhalter 1 relativ zu dem Gehäuse 2 in die distale Richtung verschiebbar ist, wenn der Spritzenhalter 1 an der Halteschulter 2e anliegt. Dies gilt auch vorteilhaft für die beschriebenen Varianten.

### Kolbenstange mit Feder und Gewindestange

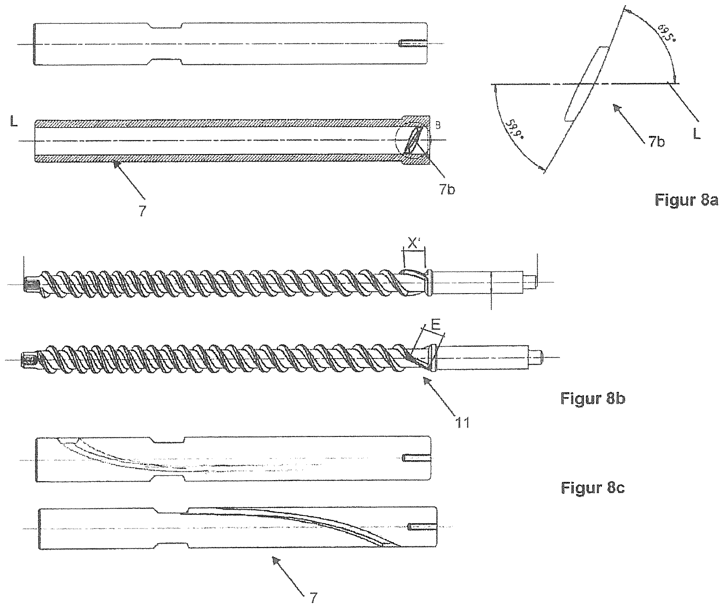

Der Autoinjektor weist ferner ein insbesondere hülsenförmiges Vortriebsglied 7 auf, welches insbesondere an seiner Innenseite einen Gewindeabschnitt insbesondere ein Gewindesegment 7b aufweist (Fig. 8a). das Gewindesegment wird in der Figur 8a in einer abgewickelten Darstellung gezeigt, wobei das Gewindesegment eine insbesondere ovale Form aufweist.

Vorzugsweise kann eine Flanke des Gewindesegments 7b des Vortriebsglieds 7 verschiedene Steigungswinkel aufweisen.

Wobei bei variabler Gewindesteigung jeweils ein anderer Bereich des Gewindesegments7b vom Gewinde insbesondere einer Gewindestange 11 berührt wird,

Insbesondere wir bei einer Schraubbewegung zwischen der Gewindestange 11 und dem mindestens einem Gewindesegment 7b des Vortriebsglieds 7, die Flanke des mindestens einem Gewindesegments 7b an dem Gewinde mit der variablen Steigung der Gewindestange geschraubt, wobei unterschiedliche Bereiche der Flanke das Gewinde mit der variablen Steigung berühren

Das Vortriebsglied ist insbesondere rotativ fest in Bezug auf das Gehäuse.

In der Figur 8c sind weitere bevorzugte Ausführungsformen dargestellt. Wie bereits erwähnt kann das Vortriebsglied 7 eine Gewindeverbindung zum Gehäuse oder einem gehäusefesten Element insbesondere dem Mechanikalter aufweisen.

Das Gewinde an dem Vortriebsglied 7 kann eine progressive oder degressive Steigung aufweisen. Abhängig davon welches Profil für den Verlauf der Ausschüttkraft erwünscht ist und welche Steigung das Gewinde am Rotationsglied 11 aufweist, wird eine dementsprechende Steigung am Vortriebsglied 7 gewählt.

Bei einem Profil bei der die Ausschüttkraft konstant sein soll und bei einer kleinen und konstanten Gewindesteigung am Rotationsglied 11, weist das Vortriebsglied eine degressive Steigung auf. D. h. für den Anfangsbereich der Ausschüttung eine kleine Steigung und gegen Ende eine grosse Steigung.

Des Weiteren umfasst der Autoinjektor wie bereits erwähnt ein Rotationsglied (11 insbesondere eine Gewindestange 11 (Fig. 8b), dessen Drehung bewirkt, dass die Federenergie an das Vortriebsglied 7 abgegeben wird, wodurch das Vortriebsglied 7 insbesondere durch einen Gewindetrieb in distale Richtung bewegt wird.

Die Gewindestange 11 ist mit einer ersten Feder 9 verbunden, die die zur Produktausschüttung notwendige Energie speichert und bei Bedarf abgibt. Die Gewindestange 11 ist mit einem Ende der ersten Feder 9 gekoppelt, wobei das andere Ende der ersten Feder 9 mit der Verschlusskappe 12 verbunden ist.

Die Gewindestange 11 weist ein Gewinde mit einer variablen Steigung auf, wobei in einem ersten Bereich das Gewinde eine grosse Steigung aufweist, Zwischen der Kolbenstange und dem Kolben ist ein Abstand bzw. Beschleunigungsweg x. Um die Beschleunigung der Kolbenstange auf dem Beschleunigungsweg x zu kontrollieren bzw. abzubremsen, und das Glasbruchrisiko zu minimieren, ist bevorzugt für den Anfang der Kolbenstangenbewegung ein GewindeeinlaufwegE mit einer grossen Steigung auf der Gewindestange 11 vorgesehen, Bevorzugt ist der Axialabschnitt x' des Gewindeeinlaufwegs E grösser als der Beschleunigungsweg x. Des Weiteren können in einer Lagerposition die Axialkräfte, welche insbesondere durch die Gewindeübersetzung aus dem Drehmoment der Feder entstehen durch eine grosse Steigung an der Gewindestange 11 gering gehalten werden.

Für ein Ausschüttprofil mit einer konstanten Ausschüttkraft variiert das Gewinde bzw. die Gewindesteigung über die Länge der Gewindestange 11.

Die Steigung ist degressiv und weist eine immer kleiner werdende Steigung auf, wodurch der Abfall des Federdrehmoments während dem Ausschütten kompensiert werden kann. Wobei die grösste Gewindesteigung im Bereich E nicht selbsthemmend ist.

Die Gewindestange 11 ist axial fest in Bezug auf das Gehäuse 2 und kann sich zumindest in eine, vorzugsweise in distaler Richtung axialfest an dem Mechanikhalter 5 abstützen

Durch die Freigabe des Vortriebsglieds 7 wird der ersten Feder 9 erlaubt, dass sie das Vortriebsglied 7 in distale Richtung bewegt. Die erste Feder 9 ist eine spiralförmige Feder, die im Ausgangs- oder Auslieferungszustand des Autoinjektors mit so viel Energie vorgespannt ist, dass sie das in dem Produktbehälter 13 enthaltene Produkt insbesondere vollständig durch Drehen der Gewindestange 11 und mit Verschieben des Vortriebsglieds 7 um einen Ausschütthub H_{A} aus dem Produktbehälter 13 ausschütten kann. Im Auslieferungszustand der Vorrichtung besteht zwischen dem Kolben 13b und dem distalen Ende des Vortriebsglieds 7 ein Abstand, so dass das Vortriebsgtied 7 erst während der Ausführung des Ausschütthubs H_{A} an den Kolben 13b anschlägt und diesen in die Ausschüttrichtung mitnimmt.

Ferner weist der Autoinjektor ein Halteelement 6 auf, welches in diesem Beispiel zwei Arme 6c aufweist, wobei an jedem Arm 6c ein erstes Eingriffselement 6a und ein zweites Eingriffselement 6b angeordnet ist. Das erste Eingriffselement 6a weist radial zu der Längsachse L hin, wobei das zweite Eingriffselement 6b radial von der Längsachse L weg weist. Das erste Eingriffselement 6a greift in eine Ausnehmung 7a, die von dem Vortriebselement 7 gebildet wird, ein, wodurch eine Bewegung des Vortriebsglieds 7 relativ zu dem Halteelement 6 in die distale Richtung oder in die Ausschüttrichtung verhindert wird. Hierdurch wird die erste Feder 9 in ihrem gespannten Zustand gehalten.

Der Autoinjektor weist ein Schaltmodul 8, 15 auf, welches eine Schalthülse 15 und eine von der Schalthülse 15 umgebene Sperrhülse 8 aufweist. Im Auslieferungszustand der Vorrichtung wird das erste Eingriffselement 6a von dem Innenumfang der Sperrhülse 8, der an dem zweiten Eingriffselement 6b anliegt, in dem Eingriff mit der Ausnehmung 7a gehalten.

Die Schalthülse 15 ist mit dem proximalen Ende 3a der Nadelschutzhülse 3 verbunden oder liegt zumindest an dem proximalen Ende 3a der Nadelschutzhülse 3 an. Eine zweite Feder 10, die vorzugsweise die Schalthülse 15 und die Sperrhülse 8 zumindest teilweise umgibt, stützt sich mit ihrem distalen Ende an der Schalthülse 15 ab. Ein Teil der Schalthülse 15 ist somit zwischen der Nadelschutzhülse 3 und dem distalen Ende der zweiten Feder 10 angeordnet. Die zweite Feder 10 ist eine als Druckfeder wirkende und als Wendelfeder ausgestaltete Feder aus Metall. Die zweite Feder 10 stützt sich mit ihrem proximalen Ende an dem Halteelement 6, insbesondere an einer Abragung 6e, die axial verschiebbar und verdrehfest in das Gehäuse 2 eingreift ab, Die zweite Feder 10 umgibt somit auch den Mechanikhalter 4 zumindest teilweise, vorzugsweise vollständig.

Das Schaltglied 15 weist eine Ausnehmung 15a auf, in welche ein Verriegelungsglied 8a der Sperrhülse 8 eingreift. Das Verriegelungsglied 8a ist sägezahnförmig und ragt radial von der Längsachse L weg. Das Verriegelungsglied 8a ist federnd an einem Arm, welcher von der Sperrhülse 8 gebildet wird, angeordnet. Durch Verschieben der Schalthülse 15 in die proximale Richtung wird die Sperrhülse 8 über den Eingriff des Verriegelungsglieds 8a in die proximale Richtung mitgenommen.

Durch Verschieben der Nadelschutzhülse 3 in die betätigte Position wird die Schalthülse 15 ebenfalls um den Betätigungshub H_{B} mitgenommen, wodurch die zweite Feder 10 gespannt wird. Wird die Nadelschutzhülse 3 nicht vollständig in die betätigte Position verschoben, kann die zweite Feder 10 die Schalthülse 15 und die Nadelschutzhülse 3 wieder zurück in die Ausgangsposition verschieben, wobei über den Eingriff des Verriegelungsglieds 8a auch die Sperrhülse 8 von der Schalthülse 15 mitgenommen wird.

Zur Verabreichung des Produkts aus dem Produktbehälter 13 wird die Abziehkappe 4 von dem Autoinjektor zusammen mit dem rigid needle shield 14 entfernt. Das distale Ende der Nadelschutzhülse 3 wird an die Einstichstelle eines Patienten angesetzt, wobei das Gehäuse 2 zu der Einstichstelle hin verschoben wird, wodurch sich die Nadelschutzhülse 3 aus ihrer Ausgangsposition um den Betätigungshub H_{B} in die proximale Richtung relativ zu dem Gehäuse 2 in die betätigte Position bewegt. Hierdurch wird die zweite Feder 10 gespannt, wobei die Schalthülse 15 von der Nadelschutzhülse 3 um den Betätigungshub H_{B} mitgenommen wird. Die Sperrhülse 8 weist eine Ausnehmung bzw. ein distales Ende 8b auf, die durch Verschieben der Sperrhülse 8 um den Betätigungshub H_{B} entlang der Längsachse L auf die Position des zweiten Eingriffselements 6b gebracht wird, wie in den Figuren 3a-3b dargestellt. Hierdurch wird das erste Eingriffselement 6a aus dem Eingriff mit dem Vortriebsglied 7 mit einer Bewegung quer zu und von der Längsachse L weg bewegt, wobei gleichzeitig das zweite Eingriffselement 6b in den Eingriff mit der Sperrhülse 8, insbesondere deren Ausnehmung 8b bewegt wird. Hierdurch wird das Vortriebsglied 7 für die Bewegung um den Ausschütthub H_{A} in die Ausschüttrichtung freigegeben.

Da die axialfeste Kopplung zwischen dem Vortriebsglied 7 und dem Halteelement 6 nun aufgehoben ist, kann das Halteelement 6, welches zumindest ein Stück weit relativ zu dem Gehäuse 2 und entlang der Längsachse L bewegbar ist, von der zweiten Feder 10 in die proximale Richtung bewegt werden, wobei das Halteelement 6 über den Eingriff des zweiten Eingriffselements 6b in die Ausnehmung 8b die Sperrhülse 8 um einen Startsignalhub Hs (Figur 3b) mitnimmt, wodurch die Sperrhülse 8 an einem Startsignalanschlag 5a, der von dem Mechanikhalter 5 gebildet wird, anschlägt und hierdurch ein akustisches und/oder taktiles Signal ausgibt, welches dem Anwender der Vorrichtung signalisiert, dass die Produktausschüttung gestartet wurde. Durch die Verschiebung der Sperrhülse 8 um den Betätigungshub H_{B} wird das Verriegelungsglied 8a und somit auch ein Sperrarm 8c, der an dem federnden Arm der Sperrhülse angebracht ist und sägezahnförmig radial zu der Längsachse L hin ragt, für eine Bewegung quer und zu der Längsachse L hin freigegeben, da der Mechanikhalter 5 eine Vertiefung 5d aufweist, welche eine solche Bewegung des Verriegelungsglieds 8a zulässt, wenn die Sperrhülse 8 um den Betätigungshub H_{B} verschoben wurde oder wenn die Nadelschutzhülse 3 in ihrer betätigten Position ist.

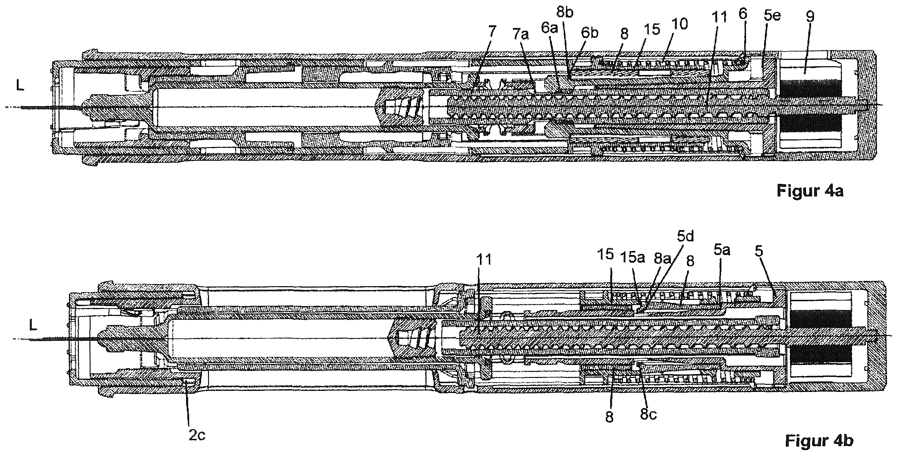

Da das zweite Eingriffsglied 6b immer noch in der Ausnehmung 8b der Sperrhülse 8 ansteht, wird hierdurch das Haltelement 6 daran gehindert, sich weiter in die proximale Richtung relativ zu dem Gehäuse 2 oder der Sperrhülse 8 zu bewegen. Das zweite Eingriffsglied 6b wird von dem Außenumfang des Vortriebsglieds 7 in den Eingriff mit der Ausnehmung 8b gehalten (Figur 4a), wenn das Vortriebsglied 7 um seinen Ausschütthubs H_{A} bewegt wird.

Am Ende des Ausschütthubs H_{A} gibt das Vortriebsglied 7 das erste Eingriffsglied 6a für eine Bewegung insbesondere zur Längsachse L hin frei, wodurch das zweite Eingriffsglied 6b aus dem Eingriff mit der Ausnehmung 8b der Sperrhülse 8 bewegt wird, so dass die zweite Feder 10 das Halteelement 6 entgegen die Ausschüttrichtung, d. h. in die proximale Richtung beschleunigt, so dass beim Auftreffen des Halteelements 6 auf dem Endsignalanschlag 5e ein akustisches und/oder taktiles Signal erzeugt wird.

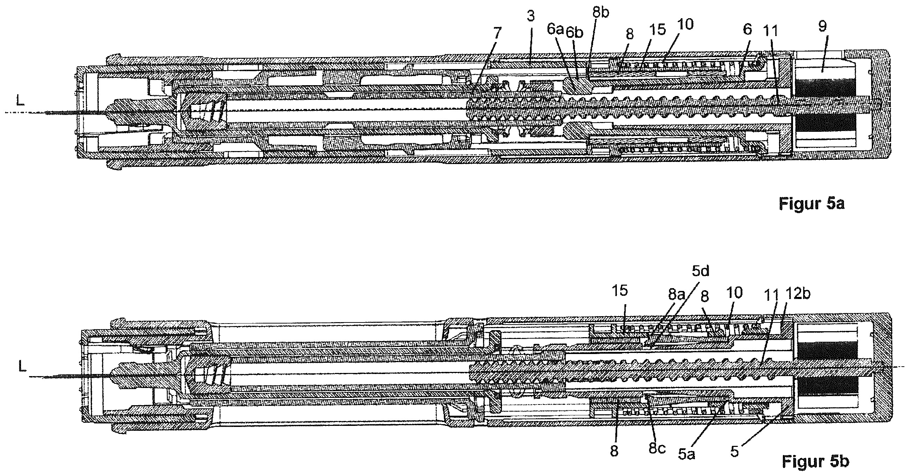

Wie am besten aus Figur 5b erkennbar ist, bleibt der Eingriff des Sperrarms 8c in die erste Ausnehmung 5d vom Mechanikhalter bestehen, wodurch eine Bewegung der Sperrhülse 8 in die distale Richtung relativ zu dem Gehäuse 2 verhindert wird.

Durch Abnehmen des Autoinjektors von der Injektionsstelle kann die zweite Feder 10 die Schalthülse 15 und die Nadelschutzhülse 3 aus der betätigten Position in die Nadelschutzposition um den Nadelschutzhub H_{N} bewegen (Fig. 7a und 7b), wobei das Verriegelungsglied 8a aus dem Eingriff mit der Ausnehmung 15a gedrückt wird, wobei sich die Schalthülse 15 relativ zu der Sperrhülse 8 in die distale Richtung bewegt. Wenn die Nadelschutzhülse 3 in ihrer Nadelschutzposition ist, verschnappt das Verriegelungsglied 8a mit der Schalthülse 15, wobei das Verriegelungsglied 8a ein erneutes Zurückschieben der Nadelschutzhülse 3 in ihre betätigte Position verhindert. Bei dem Versuch, die Nadelschutzhülse 3 aus der Nadelschutzposition in die betätigte Position zurückzuschieben, stößt das Schaltglied 15 an dem Verriegelungsglied 8a an, welches die Bewegung der Nadelschutzhülse 3 in die betätigte Position verhindert. Die Sperrhülse 8 stützt sich hierzu axial an dem Startsignalanschlag 5a des Mechanikhalters 5 ab.

**Bezugszeichenliste**

| | | | |
|---|---|---|---|
| 1 | Spritzenhalter | 13a | Nadel |
| 1a | Abragung/ Halteglied | 13b | Kolben |
| 1b | Schulter/ Eingriffsglied | | |
| 1c | Nocken | 14 | rigid needle shield/ Nadelschutzkappe |
| | | | |
| 2 | Gehäuse | 15 | Schalthülse |
| 2a | Ausnehmung | 15a | Ausnehmung |
| 2b | Ringabschnitt/Halteabschnitt | | |
| 2c | Betätigungsanschlag | H_{A} | Ausschütthub |
| 2e | Halteschulter | H_{B} | Betätigungshub |
| | | H_{S} | Startsignalhub |
| 3 | Nadelschutzhülse | H_{N} | Nadelschutzhub |
| 3a | proximales Ende | | |
| 3b | Schnappgeometrie | L | Längsachse |
| | | E | Gewindeeinlaufweg |
| 4 | Abziehkappe | x | Beschleunigungsweg |
| 4a | Schnapphaken | x' | Axialabschnitt |
| 4b | Schnapper | | |
| | | | |
| 5 | Mechanikhalter | | |
| 5a | Startsignalanschlag | | |
| 5c | Haltefederabschnitt | | |
| 5d | Vertiefung | | |
| 5e | Endsignalanschlag | | |
| | | | |
| 6 | Halteelement | | |
| 6a | erstes Eingriffselement glied | | |
| 6b | zweites Eingriffselement/ glied | | |
| 6c | Arm | | |
| 6e | Abragung | | |
| | | | |
| 7 | Vortriebsglied | | |
| 7a | Ausnehmung | | |
| 7b | Gewindesegment | | |
| | | | |
| 8 | Sperrhülse | | |
| 8a | Verriegelungsglied | | |
| 8b | erste Ausnehmung / distales Ende | | |
| 8c | Sperrarm | | |
| | | | |
| 9 | erste Feder/Ausschüttfeder | | |
| 10 | zweite Feder/Nadelschutzfeder | | |
| | | | |
| 11 | Gewindestange / Rotationsglied | | |
| | | | |
| 12 | Verschlusskappe | | |
| 12a | Rastglied | | |
| 13 | Produktbehälter/Spritze | | |

## Patentansprüche

1. Autoinjektor zur Ausschüttung eines flüssigen Produkts, insbesondere eines Medikaments,
umfassend:
a) ein Gehäuse (2) und einen in dem Gehäuse (2) angeordneten Produktbehälter (13), der einen verschiebbaren Kolben (13b) aufweist, wobei der Kolben (13b) zur Ausschüttung des in dem Produktbehälter (13) enthaltenen Produkts in eine Ausschüttrichtung verschiebbar ist,
b) ein Vortriebsglied (7), das während der Produktausschüttung auf den Kolben (13b) wirkt, und eine erste Feder (9), die vorgespannt ist, damit das Produkt aus dem Produktbehälter (13) durch Verschieben des Vortriebsglieds (7) und des Kolbens (13b) ausgeschüttet werden kann,
**gekennzeichnet durch**
c) ein Rotationsglied (11), welches operativ mit dem Vortriebsglied (7) gekoppelt ist, wobei die erste Feder (9) so auf das Rotationsglied (11) wirkt, dass zur Produktausschüttung das Rotationsglied (11) in Rotation versetzt wird, wobei das Rotationsglied (11) oder das Vortriebsglied (7) ein Gewinde mit einer variablen Steigung aufweist.

2. Autoinjektor nach Anspruch 1, **dadurch gekennzeichnet, dass** entweder das Vortriebsglied (7) mindestens ein Gewindesegment (7b) aufweist und mit dem Rotationsglied (11) in Gewindeverbindung ist oder das Rotationsglied (11) mindestens ein Gewindesegment aufweist und mit dem Vortriebsglied (7) in Gewindeverbindung ist.

3. Autoinjektor nach einem der vorhergehenden Ansprüche, **dadurch gekennzeichnet, dass** eine Flanke des mindestens einen Gewindesegments (7b) des Vortriebsglieds (7) oder des Rotationsglieds (11) verschiedene Steigungswinkel aufweist.

4. Autoinjektor nach einem der vorhergehenden Ansprüche, **dadurch gekennzeichnet, dass** das Rotationsglied (11) einen Gewindeeinlauf (E) mit einem Axialabschnitt (x') aufweist und zwischen dem Vortriebsglied (7) und dem Kolben (13b) ein Abstand insbesondere ein Beschleunigungsweg (x) ist, wobei der Axialabschnitt (x') grösser als der Beschleunigungsweg (x) ist.

5. Autoinjektor nach Anspruch 1, **dadurch gekennzeichnet, dass** das Gewinde mit der variablen Steigung, zumindest einen Bereich mit einer stetigen Steigungsvariation aufweist oder/ und die Steigungsvariation des Gewindes mit der variablen Steigung zumindest bereichsweise unstetig verläuft.

6. Autoinjektor nach einem der vorhergehenden Ansprüche, **dadurch gekennzeichnet, dass** das Gewinde mit der variablen Steigung eine degressive Gewindesteigung aufweist, wodurch der Abfall des Federdrehmoments während dem Ausschütten kompensiert werden kann.

7. Autoinjektor nach einem der vorhergehenden Ansprüche, **dadurch gekennzeichnet, dass** bei einer Schraubbewegung zwischen der Gewindestange (11) und dem mindestens einem Gewindesegment (7b) des Vortriebsglieds (7), die Flanke des mindestens einem Gewindesegments (7b) an dem Gewinde mit der variablen Steigung der Gewindestange (11) geschraubt wird, wobei unterschiedliche Bereiche der Flanke das Gewinde mit der variablen Steigung berühren.

8. Autoinjektor nach einem der vorhergehenden Ansprüche, **dadurch gekennzeichnet, dass** das Vortriebsglied (7) eine erste Gewindeverbindung mit dem Rotationsglied (11) und eine zweite Gewindeverbindung mit dem Gehäuse (2) oder einem gehäusefesten Element aufweist.

9. Autoinjektor nach einem der vorhergehenden Ansprüche, **dadurch gekennzeichnet, dass** die erste Feder (9) eine Spiralfeder ist.

10. Autoinjektor nach einem der vorhergehenden Ansprüche, **dadurch gekennzeichnet, dass** die Gewindestange (11) axialfest in dem Gehäuse gelagert ist und so mit der ersten Feder (9) gekoppelt ist, dass ein Entspannen der ersten Feder (9) zur Rotation der Gewindestange (11) führt und dass das Vortriebsglied (7) rotationsfest gegenüber dem Gehäuse (2) ist.

11. Autoinjektor nach einem der vorhergehenden Ansprüche, **dadurch gekennzeichnet, dass** ein Halteelement (6) zumindest einen axial gerichteten Arm (6c) aufweist und an dem zumindest einen Arm (6c) ein erstes Eingriffselement (6a) und ein zweites Eingriffselement (6b) angebracht sind, und wobei das erste Eingriffselement (6a) in eine Ausnehmung (7a) des Vortriebsglieds (7) lösbar eingreift, wodurch das Vortriebsglied (7) axialfest mit dem Halteelement (6) gekoppelt ist, wobei die Kopplung zwischen dem Vortriebsglied (7) und dem Halteelement (6) gelöst ist, wenn das Halteelement (6) aus dem Eingriff mit dem Vortriebsglied (7) ist, wobei durch den Eingriff das Vortriebsglied (7) daran gehindert wird, sich relativ zu dem Halteelement (6) in Ausschüttrichtung zu bewegen, wobei dieser Eingriff des ersten Eingriffselements (6a) für die Produktausschüttung lösbar ist, so dass die erste Feder (9) das Vortriebsglied (7) relativ zu dem Halteelement (6) in die Ausschüttrichtung antreiben kann.

12. Autoinjektor nach Anspruch 11, **dadurch gekennzeichnet dass** das Vortriebsglied (7) mittels der ersten Feder (9) relativ zu dem Halteelement (6) in die distale Richtung bewegbar ist, wenn das erste Eingriffsglied (6a) aus dem Eingriff mit dem Vortriebsglied (7) und das zweite Eingriffsglied (6b) in dem Eingriff mit der Nadelschutzhülse (3) oder einem Schaltmodul (8, 15) ist.

13. Autoinjektor nach Anspruch 11 oder 12, **dadurch gekennzeichnet, dass** das Halteelement (6) mit einem Vortriebsglied (7) und/oder mit dem Schaltmodul (8, 15) in Eingriff ist.

14. Verfahren zur Mehrfachverwendung eines Halteelements (6) in einem Autoinjektor nach einem der Ansprüche 11 -13 enthaltend zumindest zwei der folgenden Schritte:
- Halten des Vortriebsglieds (7) mittels des Halteelements (6)
- Axiale Bewegung des Halteelements (6) zur Erzeugung eines Startklicksignals
- Axiale Bewegung des Halteelements (6) zur Erzeugung eines Endklicksignals

## Claims

1. An autoinjector for dispensing a liquid product, in particular a drug, including:
a) a housing (2) and a product container (13) which is arranged in the housing (2) and which has a displaceable piston (13b), wherein the piston (13b) is displaceable in a dispensing direction to dispense the product contained in the product container (13),
b) a drive member (7) which acts on the piston (13b) during product dispensing and a first spring (9) biased so that the product can be dispensed from the product container (13) by displacement of the drive member (7) and the piston (13b),
**characterised by**
c) a rotational member (11) operatively coupled to the drive member (7), wherein the first spring (9) acts on the rotational member (11) in such a way that for product dispensing the rotational member (11) is caused to rotate, wherein the rotational member (11) or the drive member (7) has a thread of a variable pitch.

2. An autoinjector according to claim 1 **characterised in that** either the drive member (7) has at least one thread segment (7b) and has a thread connection to the rotational member (11) or the rotational member (11) has at least one thread segment and has a thread connection to the drive member (7).

3. An autoinjector according to one of the preceding claims **characterised in that** a flank of the at least one thread segment (7b) of the drive member (7) or the rotational member (11) has different pitch angles.

4. An autoinjector according to one of the preceding claims **characterised in that** the rotational member (11) has a thread entry (E) having an axial portion (x') and between the drive member (7) and the piston (13b) there is a spacing in particular an acceleration path (x), wherein the axial portion (x') is larger than the acceleration path (x).

5. An autoinjector according to claim 1 **characterised in that** the thread with the variable pitch has at least one region having a continuous pitch variation and/or the pitch variation of the thread with the variable pitch is at least region-wise discontinuous.

6. An autoinjector according to one of the preceding claims **characterised in that** the thread with the variable pitch has a degressive thread pitch whereby the decrease in the spring torque during dispensing can be compensated.

7. An autoinjector according to one of the preceding claims **characterised in that** in a screwing movement between the threaded rod (11) and the at least one thread segment (7b) of the drive member (7) the flank of the at least one thread segment (7b) is screwed on the thread with the variable pitch of the threaded rod (11), wherein different regions of the flank are in contact with the thread with the variable pitch.

8. An autoinjector according to one of the preceding claims **characterised in that** the drive member (7) has a first thread connection to the rotational member (11) and a second thread connection to the housing (2) or an element fixed with respect to the housing.

9. An autoinjector according to one of the preceding claims **characterised in that** the first spring (9) is a coil spring.

10. An autoinjector according to one of the preceding claims **characterised in that** the threaded rod (11) is mounted axially fixedly in the housing and is so coupled to the first spring (9) that relief of the first spring (9) leads to rotation of the threaded rod (11) and the drive member (7) is rotationally fixed with respect to the housing (2).

11. An autoinjector according to one of the preceding claims **characterised in that** a holding element (6) has at least one axially directed arm (6c) and mounted to the at least one arm (6c) are a first engagement element (6a) and a second engagement element (6b), and wherein the first engagement element (6a) releasably engages into a recess (7a) in the drive member (7) whereby the drive member (7) is axially fixedly coupled to the holding element (6), wherein the coupling between the drive member (7) and the holding element (6) is released when the holding element (6) is out of engagement with the drive member (7), wherein the engagement prevents the drive member (7) from moving relative to the holding element (6) in the dispensing direction, wherein said engagement of the first engagement element (6a) is releasable for product dispensing so that the first spring (9) can drive the drive member (7) in the dispensing direction relative to the holding element (6).

12. An autoinjector according to claim 11 **characterised in that** the drive member (7) is moveable in the distal direction by means of the first spring (9) relative to the holding element (6) when the first engagement member (6a) is out of engagement with the drive member (7) and the second engagement member (6b) is in engagement with the needle protection sleeve (3) or a switching module (8, 15).

13. An autoinjector according to claim 11 or claim 12 **characterised in that** the holding element (6) is in engagement with a drive member (7) and/or with the switching module (8, 15).

14. A method of multiple use of a holding element (6) in an autoinjector according to one of claims 11 to 13 including at least two of the following steps:
- holding the drive member (7) by means of the holding element (6),
- axially moving the holding element (6) to produce a start click signal, and
- axially moving the holding element (6) to produce an end click signal.

## Revendications

1. Auto-injecteur pour la distribution d'un produit fluide, en particulier d'un médicament, comprenant :
a) un boîtier (2) et un récipient de produit (13) agencé dans le boîtier (2), qui présente un piston coulissant (13b), dans lequel le piston (13b) est coulissant dans une direction de distribution pour la distribution du produit contenu dans le récipient de produit (13),
b) un organe d'avance (7), qui agit sur le piston (13b) pendant la distribution de produit, et un premier ressort (9), qui est précontraint, pour que le produit puisse être distribué du récipient de produit (13) par coulissement de l'organe d'avance (7) et du piston (13b),
**caractérisé par**
c) un organe de rotation (11), qui est couplé de manière opérationnelle à l'organe d'avance (7), dans lequel le premier ressort (9) agit sur l'organe de rotation (11) de sorte que l'organe de rotation (11) est mis en rotation pour la distribution de produit, dans lequel l'organe de rotation (11) ou l'organe d'avance (7) présente un filetage avec un pas variable.

2. Auto-injecteur selon la revendication 1, **caractérisé en ce que** soit l'organe d'avance (7) présente au moins un segment fileté (7b) et est en liaison filetée avec l'organe de rotation (11) soit l'organe de rotation (11) présente au moins un segment fileté et est en liaison filetée avec l'organe d'avance (7).

3. Auto-injecteur selon l'une quelconque des revendications précédentes, **caractérisé en ce qu'**un flanc de l'au moins un segment fileté (7b) de l'organe d'avance (7) ou de l'organe de rotation (11) présente différents angles de pas.

4. Auto-injecteur selon l'une quelconque des revendications précédentes, **caractérisé en ce que** l'organe de rotation (11) présente une entrée de filetage (E) avec une section axiale (x') et une distance en particulier un trajet d'accélération (x) est entre l'organe d'avance (7) et le piston (13b), dans lequel la section axiale (x') est supérieure au trajet d'accélération (x).

5. Auto-injecteur selon la revendication 1, **caractérisé en ce que** le filetage à pas variable présente au moins une zone avec une variation de pas constante ou/et la variation de pas du filetage à pas variable s'étend au moins par zone de manière inconstante.

6. Auto-injecteur selon l'une quelconque des revendications précédentes, **caractérisé en ce que** le filetage à pas variable présente un pas de filetage dégressif, moyennant quoi la chute du couple de ressort pendant la distribution peut être compensée.

7. Auto-injecteur selon l'une quelconque des revendications précédentes, **caractérisé en ce que** lors d'un mouvement de vissage entre la tige filetée (11) et l'au moins un segment fileté (7b) de l'organe d'avance (7), le flanc de l'au moins un segment fileté (7b) est vissé sur le filetage à pas variable de la tige filetée (11), dans lequel différentes zones du flanc touchent le filetage à pas variable.

8. Auto-injecteur selon l'une quelconque des revendications précédentes, **caractérisé en ce que** l'organe d'avance (7) présente une première liaison filetée avec l'organe de rotation (11) et une deuxième liaison filetée avec le boîtier (2) ou un élément fixé au boîtier.

9. Auto-injecteur selon l'une quelconque des revendications précédentes, **caractérisé en ce que** le premier ressort (9) est un ressort hélicoïdal.

10. Auto-injecteur selon l'une quelconque des revendications précédentes, **caractérisé en ce que** la tige filetée (11) est logée axialement solidaire dans le boîtier et est couplée au premier ressort (9) de sorte qu'une détente du premier ressort (9) conduit à la rotation de la tige filetée (11) et que l'organe d'avance (7) est solidaire en rotation par rapport au boîtier (2).

11. Auto-injecteur selon l'une quelconque des revendications précédentes, **caractérisé en ce qu'**un élément de retenue (6) présente au moins un bras dirigé axialement (6c) et un premier élément de mise en prise (6a) et un deuxième élément de mise en prise (6b) sont montés au niveau de l'au moins un bras (6c), et dans lequel le premier élément de mise en prise (6a) entre en prise de manière amovible dans un évidement (7a) de l'organe d'avance (7), moyennant quoi l'organe d'avance (7) est couplé axialement solidaire à l'élément de retenue (6), dans lequel le couplage entre l'organe d'avance (7) et l'élément de retenue (6) est amovible, lorsque l'élément de retenue (6) est hors prise avec l'organe d'avance (7), dans lequel l'organe d'avance (7) est empêché par la mise en prise de se déplacer par rapport à l'élément de retenue (6) dans la direction de distribution, dans lequel ladite mise en prise du premier élément de mise en prise (6a) est amovible pour la distribution de produit de sorte que le premier ressort (9) peut entraîner l'organe d'avance (7) par rapport à l'élément de retenue (6) dans la direction de distribution.

12. Auto-injecteur selon la revendication 11, **caractérisé en ce que** l'organe d'avance (7) est mobile par rapport à l'élément de retenue (6) dans la direction distale au moyen du premier ressort (9), lorsque le premier organe de mise en prise (6a) est hors prise avec l'organe d'avance (7) et le deuxième organe de mise en prise (6b) est en prise avec la gaine de protection d'aiguille (3) ou un module de commutation (8, 15).

13. Auto-injecteur selon la revendication 11 ou 12, **caractérisé en ce que** l'élément de retenue (6) est en prise avec un organe d'avance (7) et/ou avec le module de commutation (8, 15).

14. Procédé d'utilisation multiple d'un élément de retenue (6) dans un auto-injecteur selon l'une quelconque des revendications 11-13 contenant au moins deux des étapes suivantes :
- maintien de l'organe d'avance (7) au moyen de l'élément de retenue (6)
- mouvement axial de l'élément de retenue (6) pour la génération d'un signal de clic de début
- mouvement axial de l'élément de retenue (6) pour la génération d'un signal de clic de fin.
